(19) Europäisches Patentamt
European Patent Office
Office européen des brevets

(11) **EP 2 829 872 A1**

(12) **EUROPEAN PATENT APPLICATION**

(43) Date of publication:
**28.01.2015 Bulletin 2015/05**

(51) Int Cl.:
***G01N 29/24*** (2006.01)   ***G01N 29/265*** (2006.01)
***G01N 29/22*** (2006.01)   ***A61B 5/00*** (2006.01)
***G01S 15/89*** (2006.01)

(21) Application number: **13003754.2**

(22) Date of filing: **26.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Helmholtz Zentrum München Deutsches Forschungszentrum für Gesundheit und Umwelt GmbH 85764 Neuherberg (DE)**

(72) Inventors:
• **Ntziachristos, Vasilis 82166 Gräfelfing (DE)**
• **Kellnberger, Stephan 94550 Kuenzing (DE)**

(74) Representative: **Linsmeier, Josef Wallinger Ricker Schlotter Tostmann Patent- und Rechtsanwälte Zweibrückenstrasse 5-7 80331 München (DE)**

(54) **Sensor device and method for detecting thermoacoustic responses of a medium**

(57)     The invention relates to a sensor device and a corresponding method for detecting, in particular imaging, thermoacoustic responses of a medium, comprising a detector for detecting acoustic waves, in particular ultrasonic waves, generated in the medium upon absorption of energy from an electromagnetic field and/or from an alternating magnetic field comprising electromagnetic waves or magnetic waves, respectively, of a first frequency.

In order to allow for a detection of thermoacoustic responses of the medium, in particular upon absorption of energy from continuous wave (CW) electromagnetic fields and/or from alternating magnetic fields, the detector is designed for detecting acoustic waves of a second frequency which is higher than, in particular twice, the first frequency.

Fig: 4

EP 2 829 872 A1

**Description**

The present invention relates to a sensor device and a corresponding method for detecting, in particular imaging, thermoacoustic responses of a medium according to the independent claims.

Characterization of electromagnetic fields (EMFs) is important and widespread in different sectors in order to assess, for example, environmental and human exposure to non-ionizing radiation emitted by various sources, like cell phones, antennas close to residential areas or medical imaging devices. Ambient electromagnetic fields are usually measured by means of particular sensors or probes, which can be generally considered as antennas or detection coils. However, the result of such measurements usually depends on characteristics and the orientation of the probe as well as particular measurement conditions. Therefore, accurately measuring EMFs is usually a sophisticated task, both in view of sensor build-up and performance. Moreover, antennas used as measurement sensors cannot be easily inserted inside some media, for example in tissues, and, therefore, do not measure energy deposition in the medium, but only field (vector) coupling to the antenna.

In general, the interaction of transient electromagnetic fields with tissue generates ultrasonic waves through the so-called photoacoustic phenomenon. For example, ultrasonic measurements upon tissue exposure to radiofrequency (RF) or microwave sub-microsecond pulses have been employed to generate high-resolution images of electromagnetic energy deposition in tissues. The use of short RF pulses allows for a temporal separation of electromagnetic excitation and the measurement of the broadband ultrasonic tissue response as a function of time. When matter or objects are placed in the near-field of a coupling element, the use of sub-100 ns pulses becomes possible due to highly effective energy coupling vs. radiating sources so that the resolution can be further increased.

In the prior art, however, acoustic responses from gases, liquids, solid matter and/or tissues, like biological tissues or magnetic nanoparticles, have not been observed in response to constant wave (CW) electromagnetic radiation or other transient radiation other than sub-microsecond pulses.

It is an object of the invention to provide a sensor device and a corresponding method allowing for detecting, in particular imaging, thermoacoustic responses of a medium, in particular to continuous wave electromagnetic fields (EMFs), other transient EMFs and/or alternating magnetic fields.

This object is achieved by the sensor device and the method according to the independent claims.

The sensor device for detecting thermoacoustic responses of a medium according to the invention comprises a detector for detecting acoustic waves, in particular ultrasonic waves, generated in the medium upon absorption of energy from a, in particular CW or transient, electromagnetic field and/or from an alternating magnetic field comprising electromagnetic waves or magnetic waves, respectively, of a first frequency, and is characterized in that the detector is designed for detecting acoustic waves of a second frequency, wherein the second frequency is higher than the first frequency.

The method for detecting thermoacoustic responses of a medium according to the invention comprises detecting acoustic waves, in particular ultrasonic waves, generated in the medium upon absorption of energy from a, in particular CW or transient, electromagnetic field and/or from an alternating magnetic field comprising electromagnetic waves or magnetic waves, respectively, of a first frequency, and is characterized by detecting acoustic waves of a second frequency, wherein the second frequency is higher than the first frequency.

The invention is based on the newly discovered insight that the frequency of thermoacoustic responses, i.e. acoustic waves, of a medium upon excitation by transient electromagnetic fields, including alternating continuous wave (CW) electromagnetic fields, and/or alternating magnetic fields is higher than the frequency of the excitation field. In the case of a sinusoidal excitation, acoustic waves are generated at twice the first frequency (fundamental frequency) of the electromagnetic field and/or magnetic field, i.e. at the second harmonic of the first frequency. In the case of rectangular field excitation, acoustic waves are generated at the fundamental frequency, at three times the fundamental frequency, at five times the fundamental frequency and so on, i.e. they are generated at the fundamental frequency and its odd harmonics. This is in stark difference to optoacoustic responses to light excitation of a medium, where the frequency of the generated acoustic waves is the same as the frequency of the excitation pulses. This difference is assumed to be based on non-linear effects in the interaction between CW electromagnetic and/or alternating magnetic fields with matter. Accordingly, the sensor device is designed for detecting the acoustic waves at a second frequency or in a second frequency range which is above the first frequency or first frequency range, respectively, of the exciting electromagnetic and/or alternating magnetic field. In particular, the thermoacoustic responses of the medium, i.e. the acoustic waves, are detected only at the second frequency and/or in a range around the second frequency and/or in the second frequency range.

In particular, to the extent that a transient excitation can be described as a sum of sinusoids, the transient EMF or magnetic field will generate a corresponding higher-order harmonic response (second, third etc. harmonic responses), i.e. a resulting acoustic response comprising a sum of second frequencies. It is noted, however, that in the case of a transient, non-pure-sinusoidal EMF field or magnetic field, the resulting acoustic response will not necessarily contain all harmonics or be described in the sense of pure higher-order harmonic frequencies but will rather contain a mix of frequencies that depend on the nature of the transient EMF or magnetic field and possibly the sensor medium and/or objects distributed in the sensor medium (e.g. if the sensor medium is tissue or a heterogeneous medium). For example, using rectangular or triangle-shaped transient fields, only odd harmonics but no even harmonics are expected and, in particular, detected.

In summary, the invention allows for detecting, in particular imaging, thermoacoustic responses of a medium, in particular thermoacoustic responses to an excitation of the medium by transient electromagnetic fields and/or alternating magnetic fields.

According to a preferred embodiment, the second frequency of the detected acoustic waves is twice the first frequency. Accordingly, for sinusoidal electromagnetic and/or magnetic waves emitted at a certain frequency band corresponding acoustic waves are detected at double the frequency band. This may lead to an acoustic band broadening. By this means, transient electromagnetic waves at a given first frequency or frequency band can be detected with high reliability and accuracy by appropriately selecting the second frequency or second frequency band where acoustic (ultrasound) signals are collected. This embodiment is to be understood that for electromagnetic energy at a single first sinusoidal frequency, detection is preferably performed at the double frequency (second frequency). In general, for electromagnetic or magnetic fields comprising a multitude of first frequencies or a frequency band, the detector will detect preferably at second frequencies that are higher than the first frequencies. Frequency filters can be used as part of the detection process. Therefore, within the meaning of the present invention, references to "double frequency" or "second harmonic" refer to a doubling of the frequency content of the EMF and/or magnetic field frequency content for sinusoidal CW EMFs or magnetic fields or, in a more general sense, that a frequency band that is different to, in particular higher than, that of the EMF and/or magnetic field is detected. The second frequency band may contain various odd and/or even harmonics of the base frequency and/or other frequencies of the EMF and/or magnetic field. The particular content of this band will depend on several parameters including, but not limited to, the shape of the transient EMF or magnetic field.

It is, moreover, preferred that the sensitivity of the detector for acoustic waves exhibits a maximum at a center frequency which is equivalent to 1.5 to 2.5 times, in particular twice, the first frequency. Preferably, the sensitivity of the detector for acoustic waves has a broad bandwidth around the center frequency, wherein the bandwidth is equivalent to 50 % to 80 % of the center frequency or more than 80 % of the center frequency. Hereby, it is ensured that the generated acoustic waves are detected with particularly high reliability and sensitivity.

Alternatively or additionally, the detector is designed for detecting acoustic waves at a fundamental frequency of the electromagnetic field and/or of the magnetic field and/or at odd harmonics of the fundamental frequency. According to this embodiment, particularly referring to rectangular electromagnetic and/or magnetic field stimulation, the second frequency is detected at the fundamental frequency of the EMF or magnetic field and/or its odd harmonics. By this means, electromagnetic and/or magnetic fields with a rectangular field pattern can be detected using acoustic wave detectors sensitive at the fundamental frequency and its odd harmonics. The acoustic, preferentially ultrasound, wave detector can exhibit a broadband frequency response but can also have narrowband detection characteristics at the fundamental frequency and its odd harmonics. Correspondingly, the sensor device and method for detecting thermoacoustic waves can be used as a thermoacoustic spectrum analyzer. By this means, the sensor absorbs a part of the electromagnetic and/or magnetic field and generates acoustic responses, preferentially ultrasound waves, which can be captured with a detector. A spectral analysis of the detected thermoacoustic responses can be used to determine the field strength, field frequency, polarization and accordingly the field pattern. In this case, the electromagnetic and/or magnetic field does not necessarily have the pattern of a sinusoidal or rectangular pattern but can exhibit different shapes, such as sawtooth, sincs or other arbitrary waveforms.

Alternatively or additionally, the detector is designed for detecting acoustic waves at a second frequency band, wherein the second frequency band contains higher frequency components and/or harmonics of the first frequency band including the fundamental frequency.

Preferably, the detector can be a single detector, an array of detector elements or a scanning arrangement of detectors or detector elements.

According to a particularly preferred embodiment, the detector comprises an optical interferometry detector, in particular a fiber bragg grating (FBG) element or a Fabry-Perot element for detecting the acoustic waves, in particular the ultrasonic waves, by means of optical interferometry. A fiber Bragg grating is a type of a distributed Bragg reflector provided in a short segment of an optical fiber that reflects particular wavelengths of light and transmits all others. This is achieved by creating a periodic variation in the refractive index of the fiber core, which generates a wavelength-specific dielectric mirror. The FBG therefore acts as an inline optical filter or reflector which transmits or blocks certain wavelengths. Because FBGs are sensitive to strain, wherein the transmitted or reflected wavelengths depend on the applied strain, they can be used for detecting pressure variations and hence (ultra-)sound waves. Detecting the generated acoustic, in particular ultrasound, waves by means of optical interferometry and/or fiber bragg gratings allows for a particularly accurate and reliable measurement of thermoacoustic responses of the medium to a CW excitation in a broad frequency range reaching from several Kilohertz (kHz) up to several hundreds of Gigahertz (GHz), i.e. up to the microwave range.

Preferably, the fiber bragg grating element used in the optical interferometry detector being a $\pi$-phase-shifted fiber bragg grating element. Generally, an FBG is a semi-harmonic perturbation of the effective refractive index of the core of an optical fiber usually characterized by its modulation amplitude $n_{ac}$, nominal period $\Lambda$, phase $\Theta(z)$ and length $L$ in longitudinal direction $z$. An $\pi$-phase-shifted FBG is characterized by $n_{ac}(z) = n_{ac}$ and $\Theta(z) = \pi U(z - L/2)$, where $U$ is the Heaviside step function. In this case, the central frequency of the FBG is given by $v_0 = c(2n_0\Lambda)^{-1}$, wherein c and $n_0$ being the velocity of light in vacuum, and the effective refractive index of the fiber, respectively. The implementation of a FBG detector within fibers allows close proximity of the ultrasound detector to the medium measured, which is particularly advantageous for measuring EM waves of high frequency resulting in high frequency ultrasonic waves. This is because high frequency ultrasound is highly attenuated in soft tissues, water or other media and therefore propagation distances should be as short as possible.

Alternatively, instead of an FGB element the optical interferometry detector can comprise any optical resonator exhibiting a Lorenzian transmission and/or reflection spectrum, e.g. an etalon.

It is particularly preferred that the sensor device is provided with an optical interferometry detector comprising an interferometric setup with a wideband light source which generates pulsed light. Preferably, the wideband light source generates coherent pulsed light, i.e. the light source corresponds to a wideband coherent pulsed source. Preferably, the light source is a pulse laser source whose bandwidth is significantly broader than the resonance width of the optical interferometry detector. In distinction to methods, wherein the reflection and/or transmission behavior of optical resonators is interrogated by tuning a narrow linewidth laser to the resonance wavelength, the present embodiment is based on the approach to interrogate an optical resonator, like an FBG element or an etalon, by means of wideband pulse interferometry. By this means, thermoacoustic responses of media can be detected with increased sensitivity and high stability against varying environmental conditions. Moreover, pulse interferometry enables the use of passive demodulation techniques, which do not require stabilization, and performing parallel multiplexing with a single source while offering high sensitivity levels.

Alternatively, similarly preferred detector characteristics can be accomplished with Fabry-Perot interferometers optimized for high sensitivity detection or more conventional ultrasound detectors such as hydrophones and piezoelectric transducers.

A particular consideration when using non "all-optical" detectors, like hydrophones or piezoelectric transducers, is EM interference coupling to the detector. Such interference patterns are preferably eliminated for sensitive detection. Optical detectors offer natural means for reduced interference since there is no direct coupling of EM energy to silica fibers, Fabry-Perot membranes or other optical components. Nevertheless, shielding and good electrical grounding of the metallic parts of the optical-based or PZT-based detectors are preferred. Generally, when using detectors with metallic components, attention should be paid in shielding issues to avoid interference with the measured EM waves. For example, shielding approaches that are based on a metallic cover can be complemented by enclosing the entire detector into a metallic, grounded surface. Additionally, isolating transformers and/or electrically/galvanically isolated circuits, such as optocoupler circuits, can be employed to decouple and isolate the ground of the sensor and detector device from the acquisition system. It was found that the detection sensitivity of a PZT detector, for example, is not compromised when the entire detector is enclosed in a metallic encasing and/or the active detector element is covered by an aluminum foil or thin metal surface. Therefore, enclosing the detector and other electrical components into an electrically grounded membrane, typically not in contact with the detector and electric system, is a practical method to minimize interference. Detectors can be also developed with miniaturized signal digitization on or close to the active detector element and conversion to an optical signal (digital or analog) that is carried or transmitted from the enclosed transducer with a light transmission system, such as optocouplers, so as to ensure electrical isolation of the shielded sensor from its surroundings.

Such a combination of an optical detection or a PZT/electrical detection on the one hand and an optical transmission on the other hand is a preferred embodiment for minimizing interference.

Another preferred practical and powerful way to entirely eliminate interference is by means of an interrupted operation of the EM source; also termed herein quasi-CW operation. The interruption should occur at an appropriate time point which would allow detection of the ultrasonic signal in the absence of an EM wave. EM-field interruption can also be supported by damping elements introduced into the source. Thus, a fast decaying oscillation can be achieved by critical damping of the source, i.e. the EMF generating device, in order to maximize the time frame of EM-interference free detection. Ultrasound moves with the speed of sound which is much lower than the speed of EM waves. Therefore, time can be employed to also separate an EM wave from an acoustic wave. In a preferred embodiment, the EM wave is kept on for a time period. In this case, the EM wave may have the appearance of a pulse, however this pulse is not the nanosecond-range pulse typically employed in pulsed thermoacoustics, but a significantly lengthier pulse corresponding to an interrupted constant wave or transient EM wave. For optimal operation using interrupted EM waves, the design of the ultrasonic sensor should ensure that there are low capacitance and impedance effects, i.e. low EM energy storage on the metal parts and fast dissipation of any stored energy on the detector and/or on the electrical shield and/or on the RF source. Therefore, the use of a good electrical ground and the elimination of electrical and electromagnetic resistances, impedances and capacitive resistances can lead to an optimization of the time period that the EM source should be off and also enable bringing the ultrasound transducer closer to the EMF absorption region, in particular to the medium.

Preferably, a combination of shielding, low capacitance and/or optical detection can be employed to avoid EM interference onto the acoustic detector and offer highly sensitive measurements.

According to another preferred embodiment, the sensor device comprises a source for generating the electromagnetic field and/or the alternating magnetic field from which energy is absorbed by the medium. In this embodiment, both the detector and the source are components of the sensor device, which is preferably designed such that the medium to be investigated is removable, i.e. the medium can be set into the sensor device, e.g. adjacent or in proximity of the detector and/or the source, and measured and subsequently removed out of the sensor device. A source for generating alternating magnetic fields can be, e.g., a Helmholtz coil or a solenoid.

Preferably, the electromagnetic field is a radiofrequency (RF) field, in particular in the frequency range between 3 kHz and 300 GHz, especially between 0.1 MHz and 10 GHz. In this way, the presence and/or characteristics of microwaves and/or radio waves in the ambience as well as a possible interaction between these electromagnetic waves and matter, in particular biological tissue, can be detected very reliably.

The invention can be advantageously utilized for detecting and/or measuring thermoacoustic responses in a medium excited by EM continuous waves, transient waves and/or quasi-continuous wave electromagnetic fields. A continuous wave electromagnetic field comprises electrical and magnetic fields. Depending on the particular form of the source employed and/or the wave interacting with the medium, it is possible that the interaction of the tissue primarily with the electric or magnetic fields is measured. For example, tissues largely interact with the electric part of the EM field due to their conductivity and dielectric properties. However, media with magnetic particles or magnetic properties interact preferentially with the magnetic field or a combination of electric and magnetic fields. Correspondingly, the medium measured and/or the source selected may specify the particular interaction measured. For example, placement of objects in transmission or coupling elements exciting strong magnetic fields, such as closed loop elements, Helmholtz coils etc., may generate primarily a magnetic stimulation. Placement of objects in proximity with open dipoles or the far-field of transmitting antennas better stimulate strong electric responses. These schemes are only given as examples from a larger array of methods that exist in the field of electromagnetics to generate electromagnetic fields.

A quasi-continuous wave electromagnetic or magnetic field comprises electromagnetic or magnetic, respectively, waves or wavelets of constant amplitude and/or frequency within a specified time interval corresponding to the duration of the excitation of the medium. For example, a quasi-continuous wave electromagnetic field can have a fixed frequency, e.g. 2 MHz, but an amplitude exhibiting a decay, e.g. an exponential decay, over a specified excitation duration, e.g. of 10 microseconds.

Further, the invention can be advantageously utilized for detecting and/or measuring thermoacoustic responses of a medium comprising a biological tissue and/or a solid and/or a liquid and/or a mixture thereof, e.g. a suspension of solid particles in a liquid, like magnetic nanoparticles in a solvent.

Basically, the medium does not necessarily have to be an integral part of the sensor device. In this case, the acoustic detector is acoustically coupled with the medium, which represents the object under investigation the properties of which have to be determined. Accordingly, with a preferred embodiment of the invention the sensor device comprises a processing unit for deriving from the at least one detected acoustic wave one or more properties relating to the medium, in particular electric and/or dielectric and/or magnetic properties of the medium, like conductivity, permittivity and/or susceptibility.

According to another preferred embodiment, the medium is an integral component of the sensor device, i.e. the sensor device comprises both the detector and the medium. In this case, the purpose of detecting thermoacoustic responses of the medium is not primarily a determination of properties of the medium itself but rather a characterization of the electromagnetic and/or magnetic field surrounding the sensor device and penetrating the medium, like intensity, electric or magnetic field strength. Therefore, according to a preferred embodiment of the invention the sensor device comprises a processing unit for deriving from the at least one detected acoustic wave one or more properties relating to the electromagnetic field or magnetic field, respectively, in particular the strength, frequency and/or spatial distribution of the electromagnetic field or magnetic field, respectively. By this means, the detection of thermoacoustic responses of a medium according to the invention can not only be advantageously utilized for determining properties of the medium itself but also for characterizing the exciting, in particular continuous wave, electromagnetic or magnetic field.

Alternatively or additionally, the sensor device comprises a processing unit for deriving from the at least one detected acoustic wave one or more properties relating to the relaxation, absorption or deposition of energy from the electromagnetic field and/or magnetic field, respectively, in the medium. Accordingly, the detection of thermoacoustic responses of a medium according to the invention is also advantageous in view of investigating effects of the interaction between, in particular continuous wave, electromagnetic and/or magnetic fields with matter, in particular with biological tissue.

It is particularly preferred that the processing unit is designed for generating a two- and/or three-dimensional representation of the one or more properties relating to the electromagnetic and/or magnetic excitation field and/or the medium and/or the absorption or deposition of energy in the medium derived from the at least one detected acoustic wave, like a map of field parameters of the electromagnetic or magnetic field, a map of conductivity, permittivity and/or susceptibility of the medium or a map of energy absorption or deposition in the medium, respectively.

The sensor device can be also employed for generating images of transient EM energy deposition in tissue. A transient EM energy field is regarded as any EM field that is not an electric static field or a magnetic static field. So although EM pulses in the nanosecond range also qualify for detection under second harmonic detection set forth above, the invention also allows for generating such maps for any transient EM energy. To achieve this, with a preferred approach acoustic responses are obtained from different positions within and/or around the medium to be measured and combined using a processing method to generate measurements along a line, a two-dimensional image or a three-dimensional image. Possible methods to generate images include back-projection tomographic methods, delay and sum methods, raster scan methods or other image formation methods.

Such images are preferably produced over time to visualize dynamic changes of fields or medium absorption. A selection can be achieved on whether the field, the medium or their interaction is imaged. By assuming the EM field to be constant or known, the medium can be imaged. By assuming the medium to be known, the field distribution can be derived. Naturally, a combined effect of medium heterogeneity and field distribution can also be achieved. In a preferred embodiment, the field is assumed to be known and therefore the image created represents a map of energy deposition into the medium, for example tissue, i.e. characterizing properties of the medium, creating an image of the medium and/or identifying the parts that receive energy. In another embodiment, the medium is homogeneous and is used to characterize the field transmitted, emitted or coupled by the EM source.

Alternatively or additionally, the invention relates to a sensor device for measuring an electromagnetic field comprising:

- a sensor unit containing a medium which can absorb energy from the electromagnetic field and exhibits transient thermal expansion upon absorption of energy from the electromagnetic field, whereby acoustic waves, in particular ultrasonic waves, are generated, and a detector for detecting the acoustic waves and for generating according detector signals; and
- a processing unit for deriving at least one property of the electromagnetic field from the detector signals.

Alternatively or additionally, the invention further relates to a method for measuring an electromagnetic field comprising the following steps:

- providing a sensor unit into the electromagnetic field, the sensor unit comprising a medium which can absorb energy from the electromagnetic field and exhibits transient thermal expansion upon absorption of energy from the electromagnetic field, whereby acoustic waves are generated, and a detector for detecting the acoustic waves and for generating according detector signals, and
- deriving at least one property of the electromagnetic field from the detector signals.

The aforementioned sensor device and method are based on the approach to determine the presence and/or characteristics of an electromagnetic field in free space or matter, like gases, fluids, solids or tissues, by means of analyzing thermoacoustic signals, i.e. acoustic waves, generated in a sensor medium in response to an EMF energy deposition in the sensor medium and a subsequent transient thermal expansion thereof. Preferably, the sensor medium, which is placed in the EMF in free space or matter, is part of a sensor unit and can absorb at least a part of the EMF energy. For example, as a sensor medium dielectric media can be used for measurements of electrical deposition and/or magnetic media can be used for measurements of magnetic deposition. An acoustic detector, which is also part of the sensor unit, is used to record the thermoacoustic response, i.e. acoustic waves, of the excited sensor medium. The sensor unit and/or the detector can be moved relatively to the EMF and/or object under investigation in order to characterize the spatial distribution of EMFs in free space or matter or to determine an energy deposition in the object, e.g. tissue.

In distinction to direct EMF measurements via coils or antennas, by means of the invention characteristics of EMFs are measured by determining and analyzing the EMF energy absorption in the sensor medium. As a result, measuring EMFs becomes very simple and robust compared to approaches known in the art. Moreover, because the absorption of EMF energy in the sensor medium, and hence in matter, is determined directly, the invention allows for more accurate and reliable conclusions regarding the interaction between EMFs and matter and/or the energy deposited. This allows for measuring transient electromagnetic fields in a simple and reliable way.

Depending on the particular form of the medium employed for sensing purposes, different fields or interactions between the fields and the medium can be measured. For example, conductive media such as saline largely interact with the electric part of the EM field due to their conductivity. However, media with magnetic particles or magnetic properties interact preferentially with the magnetic field or exhibit a combination of electric and magnetic interactions. Accordingly, the sensor medium can be selected to differentiate between electric and magnetic interactions and separate the relative strength of the electric and magnetic fields. Conversely, in other applications it may be preferable to employ a medium that measures the total field contributions. Preferably, the medium employed as part of the sensor is selected and calibrated as to the aspect of energy to be measured.

The geometrical characteristics and the arrangement of the detector and medium can be adapted depending on the application. For example, the sound generated by the medium may be directed to a detector that is not necessarily adjacent to the medium. Such implementation may be useful in conditions of small geometries or inappropriate physical properties such as industrial measurement, measurements performed under water, heat, extreme cold conditions, and may require special protection of the detecting device. Acoustic guiding can be achieved by acoustic elements or devices, including acoustic guides or mirrors. This can result to different designs, where, e.g. the medium may be physically disconnected from the sensor. Additionally, with the use of detectors that can remotely measure sound, such as optical detectors based on interferometry, the geometrical arrangement of the sensor may comprise a medium with an impinging optical beam or a membrane and an impinging optical beam in combination with an according detector.

According to a preferred embodiment of the invention, the electromagnetic field to be measured is an electromagnetic field in free space, in fluids, like water and/or oil, or in ambient conditions, like air. Preferably, the sensor unit is placed manually or automatically into the space or ambience in which the presence and/or characteristics of electromagnetic fields have to be detected or determined, respectively. This is particularly advantageous when environmental or human exposure to generally present electromagnetic fields, e.g. emitted by cell phones or cell towers, has to be assessed.

Preferably, the sensor can be moved in a determined fashion to also capture and track the field distribution in the space and derive a map (image) of EM field absorption as a function of space co-ordinates. These maps offer an image of field distribution in the medium, i.e. field strength, when assuming that the medium measured is homogeneous. In the latter case, any modification of the signal intensity can be attributed to the variation of the field distribution. In the opposite case, where the field can be assumed to be inhomogeneous, the generated map can reveal spatial heterogeneity aspects of the medium measured. Preferably, in the most generic case, both field and medium heterogeneity can be captured. Useful information in this case can be still retrieved by assuming that either the medium heterogeneity can be otherwise estimated and used to derive the field heterogeneity. Alternatively, assuming that the field heterogeneity can be estimated, assumed or computed, this information can be used to derive the heterogeneity of the medium. This decomposition of medium heterogeneity from field heterogeneity can be accomplished by correcting the total map captured by the estimated or computed map assumed to be known. The correction may be, e.g., a division, a subtraction or the use of a more elaborate mathematical operation including data-fitting and data-modeling computation methods.

It is, moreover, preferred that the electromagnetic field to be measured is a radiofrequency (RF) field, in particular in the frequency range between 3 kHz and 300 GHz. In this way, the presence and/or characteristics of microwaves and/or radio waves in the ambience as well as a possible interaction between these electromagnetic waves and matter, in particular biological tissue, can be assessed very reliably. Due to the physical characteristics of waves in this frequency region, monitoring of particular frequencies will also determine a depth monitoring. For example, high frequency EM waves are attenuated at a higher rate with propagation distance in dissipative media. Similarly, acoustic waves can be more strongly attenuated by the propagation medium depending on the frequency of the acoustic wave. So, the frequency employed can be utilized to select the depth sampled in a resident medium or the size of the provided medium. The selection process will comprise calculating the desired signal required by the sensor and then identifying the maximum depth that such signal can be still produced.

Analysis of the frequency of the ultrasonic signal can also be used to calculate the size of the absorbers present in the medium. Absorbers of different sizes generate ultrasonic waves at different frequencies. Therefore, whereby signal strength defines the amount of absorbed energy or absorbers, a frequency analysis of the ultrasound wave can lead to the characterization not only of the distribution of the sizes of the absorbers but also the concentration or relative concentration of absorbers of different sizes. This type of analysis gives rise to the use of the sensor as a spectroscopic device on the ultrasound size; to be employed for understanding distributions of particles in matter, cells in tissue and other EMF absorbers in media. Extension of the spectroscopic principle could include using different EMF frequencies; whereby the relative energy deposition and/or the absorber size distribution can be generated as a function of EMF frequency. Essentially therefore the sensor device can be used as a "single-mode" spectroscopy sensor when either the EMF frequency is analyzed or scanned; or the ultrasonic frequency is analyzed or scanned. Alternatively, it can be used in a dual-spectroscopic-mode, whereby both EMF and ultrasonic frequencies are analyzed. Frequency scan of the EMF could be generated by changing the resonance frequency of an emitting element or by using a wideband source driven by a sweepable function generator; or generally using similar methods that can separate EMF frequencies. Frequency scan of the ultrasound signal typically implies the digitization of the ultrasonic signal using an analog-to-digital converter, using the appropriate sampling frequency, and then utilizing Fourier Transform operations to offer a frequency representation.

According to a further preferred embodiment, the at least one property of the electromagnetic field derived from the generated detector signals relates to the strength, frequency, polarization and/or spatial distribution of the electromagnetic field. For example, the frequency of the alternating electromagnetic field and/or the intensity and/or electric and/or magnetic field strength of the electromagnetic field is derived from the detector signals. Alternatively or additionally, the frequency, polarization, intensity, electric and/or magnetic field strength of the electromagnetic field is derived for a plurality of different locations in the electromagnetic field yielding a spatial distribution of the frequency, intensity, electric and/or magnetic field strength of the electromagnetic field. By this means, two- or three-dimensional maps of the obtained frequency, intensity or field strength values can be established in a reliable and simple way.

In addition or alternatively to measuring at least one property of the electromagnetic field, the processing unit can be designed for deriving from the generated detector signals at least one property of the medium of the sensor unit itself, in particular conductivity and/or a dielectric and/or a magnetic property of the medium.

Alternatively or additionally, the at least one property of the electromagnetic field derived from the generated detector signals relates to an interaction between the electromagnetic field and the medium of the sensor unit, like electromagnetic energy absorption, tissue/medium relaxation and/or deposition in the medium. For example, from the thermoacoustic signals, a two- or three-dimensional map of energy deposition in the medium of the sensor unit and/or the space in which the sensor unit is placed can be derived. In this way, further properties of the electromagnetic field regarding its interaction with matter, in particular biological tissue, can be obtained in a simple and reliable way.

In the case that the sensor is employed to measure or characterize materials, a material may be placed into the sensor arrangement, in particular adjacent or near the detector, an EMF is directed or coupled to the material, e.g. by means of a wave guide, a transmission line, a coupling element or a radiating source, and the sensor is employed to characterize aspects of the interactions, such as the EMF absorption of the material, the EMF reflection of the material or the composition of the material.

For example, the EMF source may be a frequency rich source or a source for scanning the EMF frequency to allow for spectroscopic measurements of the material and/or particles.

The medium may be a provided medium, such as a capsule of an EM absorbing substance (e.g. saline), that is inserted into the sensor unit adjacently to or in proximity of the detector. Alternatively, the medium may be a resident medium, wherein the EM energy deposition in the resident medium is measured (e.g. tissue, sea water etc.) by placing the detector of the sensor unit adjacently to or in proximity to it.

Preferably, the sensor unit comprises a medium which interacts with the electromagnetic field. In the case of dominant electric fields emitted by waveguides or dipoles or generally RF energy transmitting elements, the medium of the sensor preferably comprises conductive and/or dielectric material, for example a saline solution and/or conductive material solutions, such as carbon nanotubes, gold particles or graphene in fluid or gel. Alternatively, the sensor medium can be made of solid material, like a conductive element orsample which is inserted into the EMF to absorb a part of the electromagnetic energy. In the case of dominant magnetic field excitation employing, for example, solenoids and/or Helmholtz coils, the medium of the sensor comprises magnetically responsive material, for example magnetic fluids exhibiting super-paramagnetic, paramagnetic and/or ferro/ferrimagnetic properties. For example, the fluids may comprise particles composed of magnetite ($Fe_3O_4$ maghemite ($Fe_2O_3$), manganese ferrites, cobalt ferrites and other commercially available or existing magnetic agents which are also applied in magnetic fluid hyperthermia or magnetic resonance imaging (MRI), such as SPIOs (Endorem/Feridex), USPIOs (Combidex), MIONs or CLIOs. Particularly for the application in biological tissue, intrinsic iron storage proteins such as ferritin can be employed to sense at least one property of the electromagnetic field. In addition to magnetic fluids, the sensor medium can also comprise a conductive element or sample which is inserted into the magnetic field and which absorbs magnetic energy due to eddy current heating.

Preferably, the processing unit is designed for deriving a two- and/or three-dimensional representation of the measured property of the electromagnetic field and/or the medium, like a map of field parameters of the electromagnetic field or a map of energy absorption, tissue/medium relaxation or deposition in the medium, respectively.

Generally, in order to generate a map of electromagnetic field absorption and/or interaction with the sensor, the photoacoustic wave equation which describes the generation and propagation of acoustic waves due to absorption of electromagnetic energy can be expressed as

$$\left(\nabla^2 - \frac{1}{v_s^2}\frac{\partial^2}{\partial t^2}\right)p(\vec{r},t) = -\frac{\beta}{\kappa v_s^2}\frac{\partial^2}{\partial t^2}T(\vec{r},t) \qquad (1)$$

with the heat diffusion equation

$$\frac{1}{\alpha}\frac{\partial}{\partial t}T(\vec{r},t) - \nabla^2 T(\vec{r},t) = \frac{1}{K}Q(\vec{r},t) \qquad (2)$$

where $v_s$ is the speed of sound, $p$ is the induced pressure at position $\vec{r}$ and time $t$, $\beta$ relates to the thermal coefficient of volumetric expansion, $\alpha = \dfrac{K}{\rho C_v}$ is the thermal diffusivity with the thermal conductivity K, Q is the heating function, $\kappa = \dfrac{C_p}{\rho v_s^{\,2} C_v}$ is the isothermal compressibility with the mass density $p$, the specific heat capacities $C_p$ and $C_V$ at constant volume and pressure, respectively.

Equation 1 describes the time domain representation of the general photoacoustic wave equation. Under conditions of thermal and stress confinement, i.e. in electromagnetic environments of pulsed energy where the EM pulse is short enough so that the thermal diffusion within the tissue can be neglected, equation 1 can be further approximated by

$$\left(\nabla^2 - \frac{1}{v_s^{\,2}}\frac{\partial^2}{\partial t^2}\right)p(\vec{r},t) = -\frac{\beta}{C_p}\frac{\partial}{\partial t}Q(\vec{r},t) \qquad (3)$$

which describes the spatio-temporal relationship between thermoacoustically generated pressure $p$ and heating function Q.

Equation 3 can be solved using Greens function approach, yielding the pressure

$$p(\vec{r},t) = -\frac{\beta}{4\pi C_p}\int \frac{1}{|\vec{r}-\vec{r}'|}\frac{\partial}{\partial t}Q\left(\vec{r}',t - \frac{|\vec{r}-\vec{r}'|}{v_s}\right)d\vec{r}' \qquad (4).$$

Accordingly, the detected signal by the ultrasound sensor can be described by equation 4 in the case of pulsed excitation.

In the case of employing CW, quasi-CW or any time-extended long RF field excitation in the order of >1 $\mu$s duration, the general photoacoustic wave equation in frequency domain can be expressed as the inhomogeneous Helmholtz equation

$$\left(\nabla^2 + \frac{\omega^2}{v_s^{\,2}}\right)\hat{p}(\vec{r},\omega) = -j\omega\frac{\beta}{C_p}\left(K\nabla^2\hat{T}(\vec{r},\omega) + \hat{Q}(\vec{r},\omega)\right) \qquad (5)$$

with the fourier domain representations of the pressure $p(t)\xrightarrow{\;FT\;}\hat{p}(\omega)$, the temperature $T(t)\xrightarrow{\;FT\;}\hat{T}(\omega)$ and the heating function $Q(t)\xrightarrow{\;FT\;}\hat{Q}(\omega)$. Using the source function

$$\hat{s}(\vec{r},\omega) = K\nabla^2\hat{T}(\vec{r},\omega) + \hat{Q}(\vec{r},\omega) \qquad (6),$$

equation 5 can be rewritten in the form

$$\left( \nabla^2 + k^2 \right) \hat{p}(\vec{r},\omega) = -j\omega \frac{\beta}{C_p} \left( \hat{s}(\vec{r},\omega) \right) \qquad (7)$$

with the wave number $k = \dfrac{\omega}{v_s}$. The Green-function approach gives the solution to the Helmholtz equation 7 defined as

$$\hat{p}(\vec{r},\omega) = -\frac{j\omega\beta}{4\pi C_p} \int_V \frac{1}{|\vec{r}-\vec{r}'|} \hat{s}(\vec{r}',\omega) \exp(jk\,|\vec{r}-\vec{r}'|) d^3\vec{r}' \qquad (8).$$

To reconstruct a map of EM absorption and/or interaction with the sensor unit, the measurement and detection geometry is preferably to be considered for quantitative image representation. A preferred approach is to project the measured pressure waves on a virtual image plane or volume (back-projection reconstruction), to perform beam forming in the case of phased ultrasound arrays for acoustic wave detection (delay and sum) or to use Model-based reconstructions. This approach is typically performed by scanning the sensor and detecting multiple pressure waves at multiple locations on the surface or some distance from the medium of object scanned or the medium employed by the sensor. Alternatively, multiple such sensors can be employed in an array or other arrangement which can be static or scanned to collect the pressure waves in response to EMF absorption.

In frequency domain, i.e. when using CW, quasi-CW sources or generally time-extended EM-field stimulation, a representation of EM energy absorption/interaction with the sensor unit, in particular the medium, can be reconstructed using correlation methods, model based approaches, Finite-Element Methods (FEM) or Fourier diffraction algorithms to solve equation 8 for a two/three dimensional source distribution.

Preferably, the sensor unit is moveable relative to the electromagnetic field. In particular, the sensor unit is dimensioned and/or designed for being manually or automatically moved relatively to the electromagnetic field. The relative movement can be a free three-dimensional movement or a movement along only one, only two or three dimensions. It can be a predetermined movement or a tracked movement. Tracking may comprise optical tracking, radiofrequency tracking, magnetic tracking or other tracking methods. This is a very simple but yet reliable way for a spatially resolved measurement of properties of the electromagnetic field in free space, ambience and/or matter, like tissue.

Alternatively or additionally, it is preferred to provide a moving unit for moving the sensor unit relative to the electromagnetic field, wherein the detector is consecutively placed into multiple positions in the electromagnetic field to be measured. By this means, spatially resolved properties of the electromagnetic field in free space, ambience or matter can be determined with particularly high accurateness.

According to another preferred embodiment, the detector is designed for detecting acoustic waves and for generating according detector signals at each of multiple positions while the sensor unit is moved relative to the electromagnetic field. At each of the multiple positions at least one property of the electromagnetic field is derived from detector signals obtained for the respective position. Accordingly, the at least one property of the electromagnetic field is determined in dependence of the position, i.e. spatially resolved, in a simple, accurate and reliable way. This mode of operation may be implemented in a fast moving pattern, either continuous or oscillatory in movement, whereby the movement itself generates transient EM fields which can be detected by the sensor. This can be employed even in detecting static gradient fields, wherein the movement can track changes of the gradient and sense the field in the context of energy absorption change as a function of the movement itself.

According to an embodiment allowing for a very simple and robust sensor build-up, the detector comprises a single detector element, e.g. a piezoelectric detector element or an optical interferometry detector, having only one area sensitive for ultrasound waves.

Alternatively or additionally, the detector is designed for generating multiple detector signals upon detection of acoustic waves generated in the medium upon absorption of energy of the electromagnetic field at multiple positions. In other words, the detector is designed as a spatially resolving detector allowing for a simultaneous detection of acoustic waves at different positions and for generating according detector signals. For example, the detector comprises a one- or two-dimensional array of detector elements, e.g. piezoelectric detector elements or optical interferometry detector elements, which detect acoustic waves at different locations. By this means, spatially resolved properties of the electromagnetic field can be determined in a particularly reliable and simple way.

Preferably, acoustic coupling between the detector and the medium is achieved by ultrasound transmission fluids, such as acoustic gels or water. In the case of single element acoustic transducer and/or multiple acoustic wave detectors set in a 2D/3D array, acoustic waves can be coupled by a direct contact between the detector and the medium.

According to a further preferred embodiment, the material and/or dimensions of the sensor unit, i.e. the medium and/or the detector, are chosen such that the electromagnetic field is not significantly influenced or perturbed by the presence of the sensor unit. Preferably, the dimensions of the sensor unit, i.e. the medium and/or the detector, are significantly smaller than the dimensions of the space in which the electromagnetic field is to be measured. Preferably, the material of the medium is chosen such that it absorbs at least a part of the electromagnetic field and generates acoustic waves upon at least partial absorption of the electromagnetic field. For example, the sensor medium can be a material with dielectric and/or magnetic properties which exhibits, e.g., a certain conductivity, permittivity and/or susceptibility. The sensor medium can comprise, for example, a saline solution, a fluid of carbon and/or conductive particles, preferably micro and/or nano particles, a solution of magnetic particles or biological tissue. On the other hand, the dimensions of the medium are small enough such that the electromagnetic field is not too strongly influenced by the presence of the medium. Likewise, the detector of the sensor unit efficiently detects acoustic waves generated in the medium but is - on the other hand - only minimally invasive to the electromagnetic field. For example, the acoustic detector is a non-metallic detector and/or a piezoelectric detector and/or an optical interferometry detector which can detect acoustic waves. All in all, each of the embodiments set forth above - alone or in combination - further contributes to an even more accurate and reliable measurement of properties of electromagnetic fields.

Basically, by means of the sensor device and method described herein, properties of various kinds of pulsed electromagnetic fields and/or amplitude (AM) or frequency modulated (FM) electromagnetic fields can be measured with high accuracy and reliability. These measurements can be performed by placing or scanning the sensor close to an antenna, at a distance away loaded on a stationary or moving vehicle or employed to characterize the energy distribution or absorption by different media and matter.

The invention can be advantageously utilized for measuring so-called continuous wave (CW) electromagnetic fields of constant amplitude and frequency. Accordingly, the sensor device and method is adapted for measuring continuous electromagnetic waves of basically constant amplitude and frequency. Particularly, absorption/interaction of magnetic, electromagnetic or RF waves of a first frequency will induce a narrowband acoustic response in the medium due to thermoelastic expansion occurring at a second frequency which is higher than the first RF-wave frequency.

The induction of such non-linear acoustic waves can be described by the heating function Q in the general photoacoustic wave equation 1 and 5, respectively. In the case of RF, the heating function can be expressed as

$$\hat{Q}_{RF}\left(\vec{r},\omega\right) = \sigma\left(\vec{r}\right)\left|\hat{E}\left(\vec{r},\omega\right)\right|^2 + \pi f \varepsilon''\left(\vec{r}\right)\left|\hat{E}\left(\vec{r},\omega\right)\right|^2 + \pi f \mu''\left(\vec{r}\right)\left|\hat{H}\left(\vec{r},\omega\right)\right|^2 \qquad (9)$$

with the spectral electric field amplitude $\hat{E}$ and the spectral magnetic field amplitude $\hat{H}$, imaginary part of the complex permittivity and permeability $\varepsilon''$ and $\mu''$, respectively and the conductivity $\sigma$. Assuming a sinusoidal electric field oscillating at frequency $\omega$, the power of the electric field in the frequency domain can be rewritten as

$$\left|\hat{E}\left(\vec{r},\omega\right)\right|^2 = \frac{\pi}{2}E_0^{\,2}\vec{e}_r\left[\delta\left(\omega - 2\omega_0\right) + \delta\left(\omega + 2\omega_0\right) + 2\delta\left(\omega\right)\right] \qquad (10).$$

Substituting equation 10 into equation 9 gives the non-linear dependence between RF excitation at $\omega$ and thermoacoustic wave induced at different frequency as a function of $2\omega$. It has to be noted that a sinusoidal magnetic field oscillating at frequency $\omega$ similarly induces a non-linear acoustic wave at a different frequency $2\omega$.

Furthermore, it has to be noted that the generation and correspondingly detection of non-linear acoustic waves is not limited to the 2nd harmonic of the excitation wave, but is generally depending on the waveform pattern of the applied RF field. For example, using RF fields exhibiting a rectangular (RECT) pattern, higher harmonics will be induced at the odd harmonics of the first frequency, therefore inducing acoustic waves at n times the RF frequency with $n = 2m-1$, $m \in \mathbb{N}$, i.e. $m$ is a natural number. For instance, a rectangular electromagnetic field at frequency $f_{RF}$ stimulates acoustic waves at frequencies $f_{acoustic} = f_{RF}$, $3f_{RF}$, $5f_{RF}$, ..., i.e., the first frequency (fundamental frequency) and the odd harmonics of the fundamental frequency.

Generally, the detector is designed to capture a thermoacoustic wave due to RF stimulation of a first frequency $f_{RF}$, while the generated non-linear acoustic waves are induced at frequencies $n \times f_{RF}$ with $n \in \mathbb{N}$, i.e. $n$ is a natural number. For the case of sinusoidal electromagnetic field stimulation at frequency $f_{RF}$, a thermoacoustic pressure wave will be induced at the frequency $f_{acoustic} = 2f_{RF}$.

Accordingly, the sensor device comprises a detector that is preferably designed for detecting acoustic waves at the second harmonic frequency or at a group of the second harmonic frequencies of the EMF, i.e. acoustic waves at double the frequency of the EMF are detected by the detector, also termed "second harmonic" detection. This embodiment is based on the observation that in the case of constant EM waves or transient electromagnetic waves or fast changing electrostatic fields, the acoustic response generated by the medium resonates at double the frequency of the EM waves. Therefore, an ultrasonic detector is employed that detects acoustic waves at double the frequency or set of frequencies of the EM field to be measured. In distinction to conventional thermoacoustic imaging which requires EM pulses in the sub-microsecond pulse width range, with this embodiment EM pulses are no longer needed in order to generate thermoacoustic responses. Rather, by means of this embodiment any form of electromagnetic energy can be measured. Detection of acoustic waves at the second harmonic or double frequency of the EMF allows for a detection of signals that directly correspond to an EM energy absorption in the medium.

These second harmonic measurements can then be differentiated either in the time domain or in the frequency domain. Time domain implies a short interruption of the EM wave to eliminate EM interference to the detector element and enables the detection of an acoustic wave at a time that corresponds to the time that it takes the wave to propagate from the point of absorption to the detector. Frequency separation (frequency domain) implies the use of filters, such as electrical filters or digital, signal processing to differentiate and identify the second harmonic component.

It is to be understood that while a monochromatic (single or carrier frequency) EMF will generate an acoustic wave at double frequency, any other form of a transient EMF, for example a field with multiple frequencies, an EMF with varying some aspect of its amplitude, frequency, polarization or intensity and any other EMF energy change, will correspondingly generate a thermoacoustic response with a frequency content that is double the frequency content of the transient EMF.

As a result, the sensor device and method according to this embodiment allow for measuring any transient EM field, including fields that change any of their attributes, including constant wave EM fields, i.e. fields with an alternating intensity or frequency-modulated fields such as the ones employed by radar or radio stations etc. Moreover, it is noted that thermoacoustic waves generated in the medium upon excitation by ultra-short nanosecond pulses, which also constitute a transient, frequency rich EM field, can be also detected by means of the second harmonic frequency detection disclosed herein.

Further advantages, features and examples of the present invention will be apparent from the following description of following figures:

Fig. 1    shows a first example of a sensor device;

Fig. 2    shows a second example of a sensor device;

Fig. 3    shows a third example of a sensor device;

Fig. 4    shows examples of an RF excitation signal and a thermoacoustic response;

Fig. 5    shows examples of setups of an interferometric detector;

Fig. 6    shows a schematic representation of (a - c) narrowband interrogation (d - f), wideband CW interrogation and (g - i) wideband pulse interrogation of an optical bandpass filter;

Fig. 7    shows a further example of an RF excitation signal and a thermoacoustic response; and

Fig. 8    shows an example of a sensor unit for detecting thermoacoustic responses of a medium to alternating magnetic fields.

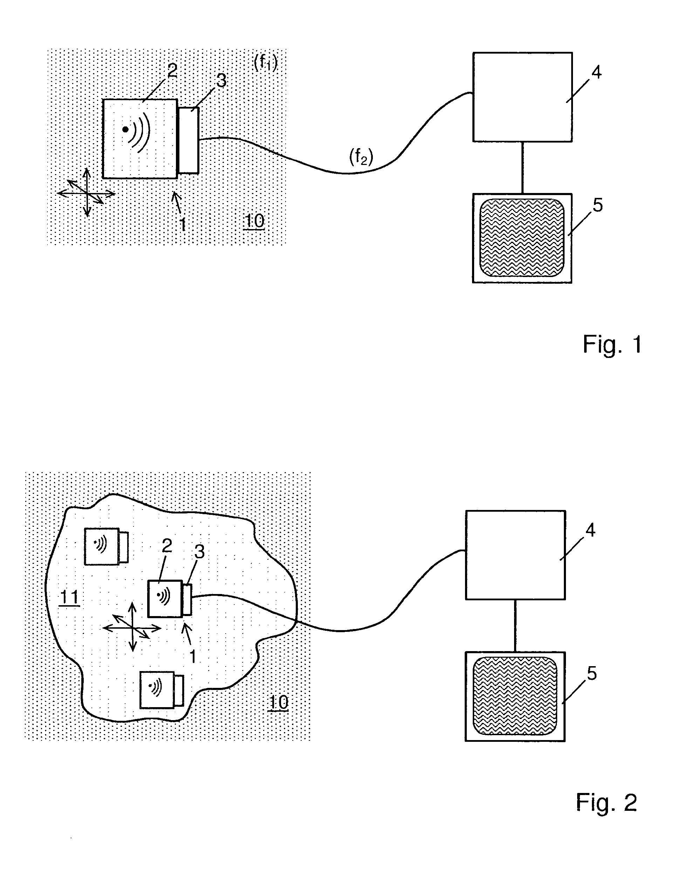

Figure 1 shows a first example of a sensor device for measuring an electromagnetic field 10 and/or for detecting thermoacoustic responses of a medium 2.

Preferably, the electromagnetic field 10 is an electromagnetic field in free space or in the ambience, like in air and/or under ambient conditions. The electromagnetic field 10 can comprise pulsed and/or amplitude modulated (AM) and/or frequency modulated (FM) electromagnetic waves but can also be a continuous wave (CW) electromagnetic field of constant amplitude and frequency. The frequency of the electromagnetic field 10 to be measured is preferably in the region of radio waves and/or microwaves, preferably between approximately 3 kHz and 300 GHz.

The sensor device comprises a sensor unit 1 with a medium 2 and a detector 3. The medium 2 is thermoacoustically sensitive to the electromagnetic field 10 surrounding the sensor unit 1. In particular, the medium 2 at least partially absorbs energy from the electromagnetic field 10 and/or interacts with the electromagnetic field 10, which penetrates into the medium 2, and shows a subsequent transient thermal expansion and contraction resulting in acoustic waves, in particular ultrasound waves, which can be detected by detector 3.

For reasons of clearness, the absorption and/or interaction of electromagnetic energy at a certain position in the medium 2 and the subsequent generation of acoustic waves is illustrated in figure 1 in a strongly simplified way by a single point and arcs partially surrounding this point. In a real medium 2, however, electromagnetic energy is absorbed at a plurality of positions within medium 2 such that acoustic waves emerge from a plurality of positions within the medium 2.

The medium 2 can comprise various materials or compositions showing a thermoacoustic effect, i.e. a generation of acoustic waves upon absorption of electromagnetic energy, like gases, liquids, solids, mixtures of liquids and solids or biological tissue.

The detector 3 can be, for example, a single piezoelectric transducer (PZT) or a one- or two-dimensional array of piezoelectric transducers (PZTs) for a spatially resolved detection of the acoustic waves. Alternatively, the detector 3 comprises an optical interferometry detector for detecting the generated acoustic waves. Preferably, the detector 3 comprises a fiber bragg grating (FBG) element, the optical transmission and/or reflection properties of which being sensitive to strain or pressure, and an optical detection element, e.g. an optical interferometer, for detecting the change of the transmission or reflection properties of the FBG due to the impinging acoustic waves.

Alternatively or additionally, the detector 3 may comprise an array of acoustic detector elements to simultaneously detect acoustic signals emerging from the medium 2 at different positions. The array of detector elements of the detector 3 can be coupled directly to the medium 2 by means of ultrasound transmission substances.

The detector 3 converts the detected acoustic waves into according detector signals which are forwarded to a processing unit 4, where at least one property of the electromagnetic field 10 is derived from the detector signals. The at least one property of the electromagnetic field 10 can be, e.g., the intensity and/or frequency and/or polarization of the electromagnetic field 10 and/or the electric and/or magnetic field strength at a certain position within the electromagnetic field 10.

Preferably, the sensor unit 1 is designed as a handheld sensor unit which can be grasped and/or manually moved relatively to the electromagnetic field 10. It is, moreover, preferred that the sensor unit 1 can be freely moved in space to any desired position in the electromagnetic field 10. This is indicated by double arrows along the three dimensions.

When detecting acoustic waves at a plurality of positions within the electromagnetic field 10, e.g. by moving the detector unit 1 relative to the electromagnetic field 10 and/or by detecting the acoustic waves with spatially resolving detectors, according detector signals and properties of the electromagnetic field 10 can be obtained for a plurality of desired positions in the electromagnetic field 10.

The derived properties of the electromagnetic field 10 are preferably displayed at display unit 5. In the case that properties of the electromagnetic field 10 are determined with spatial resolution, i.e. at a plurality of different position, a two- or three-dimensional map of these properties can be displayed at the display unit 5.

Figure 2 shows a second example of a sensor device, wherein a sensor unit 1 is positioned within an object 11 that is surrounded by an electromagnetic field 10. The object 11 can be, for example, a gas, a liquid, a solid or a tissue. Preferably, the dimensions of the sensor unit 1 are significantly, preferably more than five or ten times, smaller than the dimensions of the object 11 in order to avoid or to reduce a possible perturbation of the electromagnetic field 10 within the object 11 by the sensor unit 1.

Like with the first example described above, the medium 2 of the sensor unit 1 in the second example of fig. 2 absorbs a part of the energy of the electromagnetic field 10 penetrating the object 11, whereupon acoustic waves are generated which can be detected and converted to according detector signals by detector 3.

By moving sensor unit 1 and object 11 relatively to each other, as indicated by detector units in two further exemplary positions, acoustic waves corresponding to the electromagnetic field 10 at different positions within the object 11 are detected by detector 3 and properties of the electromagnetic field within the object 11 and/or properties of the object 11 itself, e.g. electromagnetic absorptivity, permittivity or susceptibility, can be derived from according detector signals in processing unit 5. Regarding further preferred characteristics, functionalities and advantages of sensor unit 1, medium 2, detector 3, processing unit 4 and display unit 5 the elucidations with respect to the first example set forth above apply accordingly.

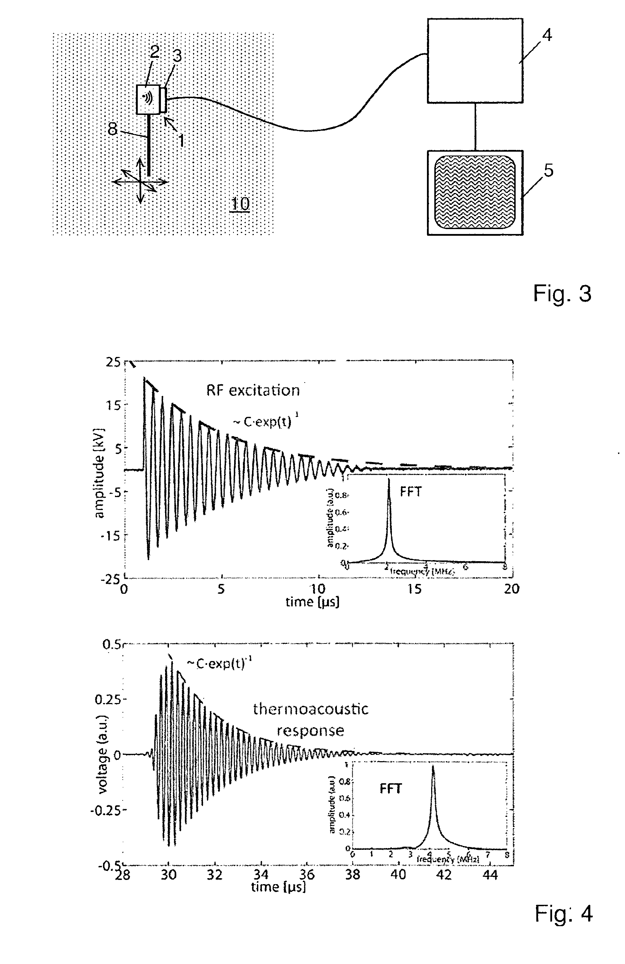

Figure 3 shows a third example of a sensor device, wherein a moving unit 8 is provided for moving the sensor unit 1 relatively to the electromagnetic field 10. The moving unit 8, which is represented only schematically, can be realized by various mechanisms, like servo motor drives and/or piezoelectric actuators etc. By means of the moving unit 8, an accurate and reliable movement and/or positioning of the sensor unit 1 to desired positions within the electromagnetic field 10 is achieved so that spatially resolved properties of the electromagnetic field 10 can be derived with an accordingly high accurateness and reliability. Regarding further preferred characteristics, functionalities and advantages of sensor unit 1, medium 2, detector 3, processing unit 4 and display unit 5 the elucidations with respect to the first example set forth above apply accordingly.

In the following, further alternative and/or additional aspects and/or advantages of the sensor device and method are specified.

The thermoacoustic sensor device and method set forth herein are preferably used for measurements of electromagnetic energy deposition in space and/or in tissues and/or measurements of the dielectric and/or conductive properties of material and/or detecting thermoacoustic responses of a medium. The method is based on resolving the thermo-elastic generation of ultrasonic waves within the sensor material, like saline solution, fluids of carbon and/or conductive (micro- and/or nano-) particles, solution of magnetic particles or tissue, produced in response to transient electromagnetic energy absorption. Therefore, it relates to a direct measurement of EMF absorption by tissue or other material, but with significantly different characteristics compared to, e.g., prior art coil measurements that attempt to measure the EMF directly. In other words, the sensor device and method relate to a direct measurement of the actual EMF absorbed by the material.

The principle of operation preferably relates to

a) moving the sensor, which contains the absorbing material, in free space and determining the field, wherein the sensor is preferably made as minimally invasive to the field as possible so as not to perturb the field significantly, or

b) engulfing the material to be measured by the detector in order to determine an energy distribution in the material itself.

Preferred components of the sensor device are as follows:

- An acoustic detector, e.g. a non-metallic sensor, preferably an interferometric optical sensor that can detect acoustic fields.
- A processing unit for converting the raw measurements from the acoustic detector to an EMF absorption measurement and for converting space measurements to maps. For example, the processing unit converts an electrical signal obtained from a piezoelectric (PZT) detector or a resonance signal obtained from an interferometric detector to the amount of energy deposited in medium.
- A display unit which displays a corresponding measurement.
- A sensor material, which is part of the sensor unit in the case (1) that the EMF has to be measured. In the case (2) that properties of the material itself are determined, the material may not necessarily be an integral or fixed component of the sensor unit but rather an exchangeable part thereof. In first case (1), the material can be a medium with dielectric and/or magnetic properties, for example a material that attains conductivity, permittivity and/or susceptibility and acts as a part of the sensor device. The material can be provided as one element or multiple elements. In second case (2), the material does not have to be part of the sensor device, as the measurement is performed directly on the material of interest.

Apart from building new knowledge on the distribution of fields within media, including air, the sensor device and method allow for a direct visualization of energy deposition, the most important parameter when addressing biological effects or characterizing, for example, MRI coils, antennas and other energy coupling or transmitting elements.

The resolution of generating EMF absorption maps can be significantly high. In first case (1) the resolution preferably relates to the size of the sensor material and the size of the steps of a translation or movement of the sensor device in space. Potentially, more than one sensor can be employed for measurements over large or small volumes in parallel. In second case (2) the resolution depends on the scanning parameters of the sensor and on the geometrical and frequency characteristics of the sensor.

For measurements of absorption distribution, for example for studying the dielectric properties of a material, unprecedented resolution of less than 5 to 100 microns can be achieved, appropriate for sensing distributions at cellular or subcellular levels, especially when high frequency acoustic detectors are employed.

An EMF source can be a mobile phone antenna, a radar emitter or a coil, for example an MRI coil, whereby characterization of the coil itself is a foreseen application. The source could be also a source developed specifically to enable thermo-acoustic measurements for absorption or dielectric property characterization, for example a radiating source or a near-field source. In all cases, detection can be achieved when there is a transient component in the source. For example, the source emits pulses or the source is of varying intensity, such as sources of a carrier frequency, amplitude modulated sources, frequency modulated and phase modulated sources etc. Typically, the EMF sources have at least one frequency component to induce a response to the sensor device. However, for sensing dielectric properties, specific sources that maximize the sensitivity of the sensor can be considered, for example EM pulses, or EM chirp modulation.

The invention addresses many different application fields. For example, electromagnetic absorption in tissues can be directly measured with near-field radiofrequency thermoacoustic (NRT) tomography in high resolution which can provide a visualization of EMF absorption at the cellular and sub-cellular levels. Second, by separately emitting electric or magnetic fields different contrast mechanisms can be imparted for measuring conductive tissue properties and magnetic molecules and nano-particles in high resolution. The sensor allows for high resolution implementations reaching to sub-10 micron resolution, preferably between 3 and 5 microns, in cells and 50 to 200 microns in big tissue volumes. Such high resolutions can be more typically achieved in above-mentioned second case (2), whereby inversion is employed after detection to reconstruct a map of energy deposition.

The sensor device can be employed to characterize the strength of an electromagnetic energy emitting, radiating or coupling element. This can be accomplished by placing the sensor unit in the vicinity of the antenna and relating the detected acoustic signal to an energy level. This can be achieved by sensor unit calibration. i.e. measurement of known energies with the sensor unit and generation of a scale that relates the energy absorbed by the medium to ultrasonic signal strength. The relation of acoustic signal to energy absorption can also be achieved by other methods such as measurement of relative changes between antennas or spaces, through different media or time and computation of energy changes. By the same means, by moving or tracking the sensor unit, or utilizing a multi-detector device, i.e. a detector containing multiple detector elements, the field of the emitting or coupling energy can be characterized. This includes identifying the directionality of the element, measuring the near-field response of the field, measuring the field distribution within or around objects or structures, for example around or within buildings.

The sensor can also be employed to measure the attenuation of EM energy from different materials, characterize materials based on their attenuation, or determine the efficiency and examine the design of EM shielding approaches or constructions.

Another alternative or additional aspect relates to a sensor device and method, wherein:

- A homogeneous medium (for example ionized water, fluids with conductive particles and/or fluids with magnetic particles) is used to calibrate the field distribution of the EM source.
- A sample of interest, such as a tissue or another material, is introduced as the medium into the sensor unit and measured by the sensor device.
- The measurements of the sample of interest are normalized by the measurements from the homogenous medium in order to obtain accurate measurement of energy absorption or of tissue dielectric properties.

Preferably, the high resolution that can be achieved makes it possible to measure not only energy deposition at the tissue level but to directly visualize the absorption from different cellular compartments at the cellular level. By coupling these measurements to fluorescence microscopy one can further correlate the findings to conventional optical microscopy to study the effect of EMF absorption on the expression and function of cells and proteins, using fluorescent protein tagging. This approach can offer insights on the fundamental interactions of tissue with EM energy. Besides cellular imaging, the sensor device can be employed to study tissues, at 50 to 200 micron resolution, using a portable scanner.

Further potential applications span from the development of tools allowing for directly measuring electromagnetic (EM) energy deposition and studying interactions with tissues and other acoustically compatible media, such as food, to sensing devices for environmental, biological and medical applications or for food inspection.

With a preferred implementation of the invention in a microscopy cellular sensor, ultra-high frequency transducers (> 50MHz) are used for cellular resolution imaging.

With yet another preferred implementation of the invention in a macroscopic sensor, a near-field thermoacoustic (NRT) tissue scanner is provided using ultrasound sensing components in the 1 to 50 MHz range.

The microscopy and tissue sensors can be also implemented as electro-acoustic or magneto-acoustic sensors, depending on the field present in the measurement (electric or magnetic) which in turn depends on the EM source employed, which could be made only magnetic, or only electric, in order to sense correspondingly electric or magnetic responses. Similarly, the sensor can be used for characterizing EM pulses, EM quasi-CW sources or CW sources.

By means of near-field thermoacoustic (NRT) imaging, the spatial resolution of the derived properties of the EMF and/or the medium, can be significantly improved. Near-field implies that the object, i.e. medium, is placed at a subwavelength distance to the coupling element, i.e. the EMF source. Operation in the near-field, although theoretically more complex, allows more efficient energy coupling than usual radiating sources and therefore can significantly minimize the cost of equipment, the source power requirements and importantly the pulsewidth (PW) employed, the latter being the major reason for the resolution improvement, since the resolution is directly proportional to the PW multiplied with the speed of sound. In particular, the efficient energy coupling in the near-field allows operation with ultra-fast pulses of preferably less than 5 ns which can lead to a resolution of less than 10 microns, possibly closer to 5 micron or better depending on the signal to noise ratio achieved, assuming that correspondingly ultra-high bandwidth ultrasound transducers are employed (> 100 MHz).

In order to avoid interference effects from EMFs during the detection process, preferably a non-metallic, Bragg grating-based interferometric detector is used which is transparent to EMFs while offering broad tunability and sensitivity that equals that of conventional piezoacoustic ultrasound detectors.

The invention can also be used to investigate new contrast mechanisms for biomedical, environmental and food sensing applications, such as the separation of electric contrast from magnetic contrast. It can therefore explore (as a single measurement or also in high resolution) "electric contrast" (i.e. conductivity, permittivity) and "magnetic contrast" foreseen here coming from resident tissue iron or iron-oxides and other magnetic particles (i.e. paramagnetic, superparamagnetic, ferromagnetic or other). These implementations are considered in the context of highly focused (i.e. high resolution) "microscopy" measurements and of macroscopic sensing.

Additional aspects of the sensor or the method according to the invention are discussed in the following.

According to a further aspect, an EM source, such as a transmission line pulse, a CW source, or a time-intermittent quasi-CW or chirp EM source can be provided as an EM source, when no external EMF is already present in the foreseen application (for example in sensing dielectric properties in tissue).

Moreover, electrically-weighted vs. magnetically-weighted excitation can be applied by utilizing tissue coupling based on dipoles or waveguides (electric) vs. Helmholtz coils/solenoids (magnetic), respectively. The coupling devices may consist of multiple elements for achieving wider-band operation over single elements.

According to another aspect, the invention can be used for investigating the sensitivity of electro-acoustic and magneto-acoustic implementations in imaging conductive and/or permittive and magnetic contrast and/or magnetic relaxation (from magnetic particles) and investigating contrast from resident tissue conductive properties, iron deposits or other, extrinsically administered superparamagnetic/paramagnetic, ferro-/ferrimagnetic and/or conductive material.

According to yet another aspect, the invention can be used for determining the EM pattern in material (possibly after calibration) for direct sensing and visualization of the field distribution in the material, or for characterization/optimization of coils, antennas and other EM sources.

Preferably, the sensor device is designed as a handheld sensor device which can be held by hand and manually moved relatively to the EMF or object under investigation, like an ultrasound scanner for point-of-care, bed-side or homecare applications, for example, in monitoring vital signs and other disease or wellbeing biomarkers.

As already indicated above, the sensor device according to the invention can be used for cellular measurements and/or for registering the obtained images to microscopy images for validation purposes and fluorescence microscopy measurements for characterization of biological EM effects.

Preferably, the sensor device according to the invention can be used for characterizing tissues, EMF heterogeneity in space or materials, coupling and radiation performance of antennas (in the near-field or far-field), measuring the delivered energy from EM sources (Radar, Mobile phones, other), or tissue parameters for risk evaluation, diagnostic or theranostic applications.

Particularly, the sensor device can be used to measure electromagnetic field distributions in different liquids, fluids and gases. For example, the sensor device can be employed for underwater EMF measurements without distorting the field homogeneity due to the small size of the sensor.

It is, moreover, preferred to design the sensor device as a small and general-use EM sensor device utilizing a saline (or similar) sensor material, such as a saline volume, and utilize a fiber-based or PZT-based acoustic sensor (the detector having size of less than 200 microns) in the saline volume as a generic sensor of EM strength and/or field frequency and/or field distribution. The sensor can then be used as direct measuring device of EM energy deposition in space.

Another advantageous application includes monitoring of benign and/or malign tissue to investigate physiological aspects, tissue growth and development on a cellular and subcellular level as well as visualization of neurological functions.

According to another aspect, the sensor unit and/or detector can be used as a theranostic tool in targeted and untargeted hyperthermic treatments as it unifies therapeutic treatment and monitoring at the same time. Magnetic fluids, exhibiting superparamagnetic, paramagnetic or ferro/ferromagnetic characteristics can be applied to a desired region, i.e. a tumor region, while treatment with CW or quasi-CW magnetic fields can be monitored by detecting the non-linear acoustic responses of the tissue. Similarly, conductive fluids, for example gold particle fluids or carbon nanotube fluids can be employed to treat a disease, for example a tumor, while the sensor unit and/or detector can be utilized to monitor the progress of treatment simultaneously.

According to another aspect, the sensor unit and/or detector can be inserted into the body, for example inside a hollow organ, the esophagus, into the colon/gastrointestinal track, or inside a blood vessel. In this case of intravascular, colonoscopic, esophagus, transurethral or cardiac imaging, the sensor medium is perfused through the region of interest, is conjugated with peptides or antibodies to bind to specific regions of tissue or is coupled to the detector while the absorbed magnetic or electromagnetic or RF energy is measured with the ultrasound detector, for example a FBG.

A further advantageous application includes thermal tissue ablation for treatment of diseases. As an example, electromagnetic energy can be applied by physically contacting the desired region by means of the sensor while the treatment, i.e. the ablation of tissue can be monitored acoustically. Additionally or alternatively, the sensor medium can be injected intravenously to target specifically desired tissue while the absorption of EM/magnetic energy can be detected with ultrasound detectors.

According to another aspect, the sensor device can be used as a flow meter to characterize quantitatively the perfusing sensor medium. In this case, the sensor medium comprises a magnetic fluid to derive at least one part of the magnetic field or a conductive fluid to derive at least one part of the electromagnetic fluid.

Last but not least, the sensor device and method can be used to characterize the presence of iron-oxides or other super-paramagnetic or ferromagnetic particles in material or tissue and/or to characterize the electromagnetic (dielectric) properties of cancer, cardiovascular disease, inflammation, rheumatoid arthritis, liver disease/diabetes. In the case of dominant electric field excitation, the sensor device and method can furthermore be used to characterize the presence and distribution of conductive particles such as carbon nanotubes or graphene in material and tissue.

In the following, further preferred embodiments of the sensor device and method will be elucidated.

As already set forth above, by means of the sensor device and the according method thermoacoustic responses, i.e. detector signals, can be obtained upon an excitation of the medium by both electromagnetic fields and transient or alternating magnetic fields. Therefore, the above elucidations with respect to electromagnetic fields apply to magnetic fields accordingly. Hence, in the elucidations given with respect to the embodiments shown in figures 1 to 3, the electromagnetic field 10 can be - alternatively or additionally - regarded as a transient and/or alternating magnetic field.

Moreover, although the sensor device shown in figures 1 to 3 preferably comprises a medium 2, the medium 2 may not necessarily be a part of the sensor unit 1. Rather, according to an alternative, but yet particularly preferred embodiment, the sensor device may comprise a detector for detecting acoustic waves generated in a medium 2 upon absorption of energy from an electromagnetic and/or alternating magnetic field. In this alternative embodiment, the medium 2 may preferably be a medium that is provided near or adjacent to the detector of the sensor device only while thermoacoustic measurements are performed.

Further, in the examples according to figures 1 to 3 a source of the electromagnetic field 10 and/or magnetic field is preferably not part of the sensor device. Nevertheless, according to an alternative, but yet particularly preferred embodiment, the sensor device may, in addition to a detector 3 and/or a medium 2, comprise a source for generating an electromagnetic field 10 and/or alternating magnetic field.

Preferably, the sensor devices shown in figures 1 to 3 comprise a detector 3 for detecting acoustic waves, in particular ultrasonic waves, generated in a medium 2 upon absorption of energy from an electromagnetic field 10 and/or alternating magnetic field 10 comprising electromagnetic waves or magnetic waves, respectively, of a first frequency $f_1$, wherein the detector 3 is designed for detecting acoustic waves of a second frequency $f_2$, wherein the second frequency $f_2$ is higher than the first frequency $f_1$, i.e. $f_2 > f_1$. Preferably, the second frequency $f_2$ of the detected acoustic waves is equivalent to 1.8 to 2.2 times the first frequency $f_1$ ($f_2 = a\, f_1$, with $a = 1.8 \ldots 2.2$), preferably twice the first frequency, i.e. $f_2 = 2f_1$.

This aspect is based on the surprising fact that the frequency $f_2$ of thermoacoustic responses, i.e. acoustic waves, of the medium 2 upon excitation by a continuous wave (CW) electromagnetic field 10 and/or alternating magnetic field 10 is approximately twice as high as the frequency $f_1$ of the excitation field 10. Accordingly, the detector 3 is designed for detecting acoustic waves, preferably only, at a second frequency $f_2$ corresponding to approximately twice the first frequency $f_1$ of the exciting CW electromagnetic and/or alternating magnetic field 10. Preferably, the detector 3 is designed for detecting acoustic waves, preferably only, in a frequency range around the second frequency $f_2$.

Figure 4 shows examples of a radiofrequency (RF) excitation signal (upper part of fig. 4) and a corresponding thermoacoustic response (lower part of fig. 4) together with respective Fourier-transformed signals (FFT). The shown thermoacoustic response was measured on a medium 2 consisting of or containing copper.

As obvious from the upper part of fig. 4, the amplitude of the electromagnetic waves of the RF excitation exhibits an exponential decay over time t, whereas the frequency of the excitation is constant. Accordingly, the Fourier-transformation FFT (see inset of upper part of fig. 4) of the RF excitation shows a finite width around a first frequency $f_1$ of approximately 2.1 MHz.

In response to an excitation with the RF electromagnetic field, acoustic waves are generated in the medium 2, detected by detector 3 and converted into according detector signals of a second frequency $f_2$. As obvious from the lower part of fig. 4, the amplitude of the detector signals, i.e. the thermoacoustic response, also shows an exponential decay over time t. In distinction to the first frequency $f_1$ of the RF excitation, however, the detector signals of the thermoacoustic response exhibit a second frequency of approximately 4.2 MHz, which corresponds to twice the first frequency $f_1$ of the RF excitation of approximately 2.1 MHz. Accordingly, the Fourier-transformation FFT of the thermoacoustic response (see inset of lower part of fig. 4) exhibits a finite width around the second frequency $f_2$.

The time-shift with respect to the RF excitation of approximately 20 $\mu$s originates from placing the detector 3 approximately 20 $\mu$s away from the medium 2 to be measured, such that possible interference signals between the excitation and the detector 3 were separated from the measurement of the ultrasonic responses. A temporal distance of 20 $\mu$s corresponds to a spatial distance between the excited between the medium and the detector of $x = v\, 20\ \mu$s, wherein $v$ is the sound velocity in air, water or any other matter between the medium and the detector.

In the example shown in fig. 4, the RF excitation decays exponentially over a period of approximately 10 $\mu$s and can therefore be regarded as a quasi- CW excitation. The sensor device and method according to the invention are, however, also suitable for detecting thermoacoustic responses upon any regular CW excitation, wherein both first

frequency $f_1$ and amplitude of the excitation field are constant over time. This applies both for electromagnetic and/or magnetic CW excitation.

Further measurements with the sensor device shown in figures 1 to 3 yield according results. For example, an RF excitation of a copper medium 2 at a first frequency $f_1$ of approximately 3.1 MHz yields a thermoacoustic response at a second frequency of approximately 6.2 MHz. Moreover, RF excitation of biological tissue, like excised pork muscle tissue, at a first frequency $f_1$ of approximately 2 MHz results in thermoacoustic responses at a second frequency $f_2$ of approximately 4 MHz.

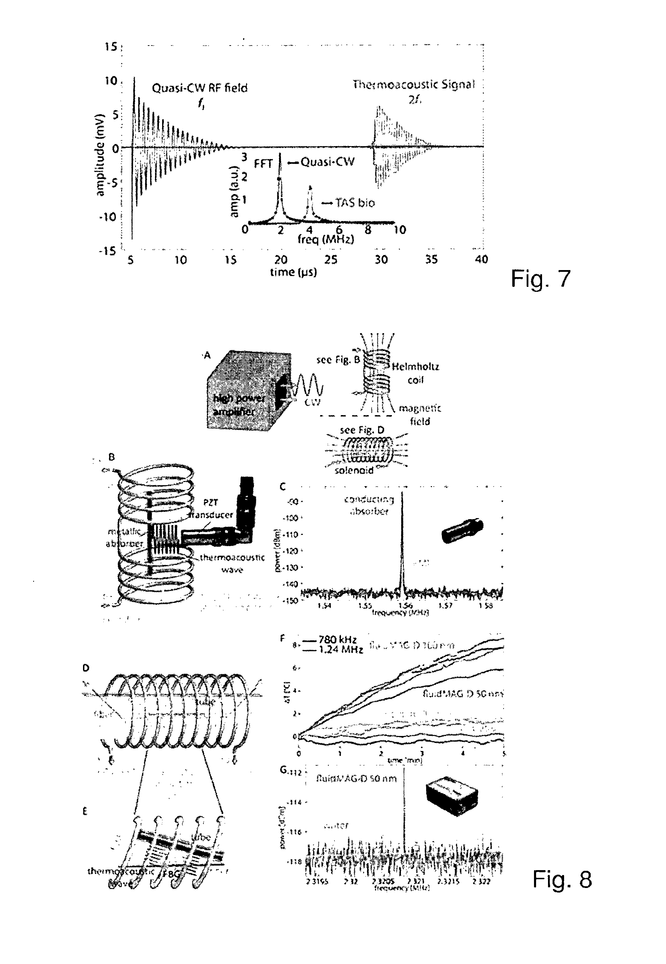

Figure 7 shows an according example of a non-linear 2nd harmonic thermoacoustic wave at a frequency $2f_1$ of approximately 4 MHz induced in biological tissue after exposure to quasi-CW RF fields of a frequency $f_1$ of approximately 2 MHz. The lines in the left part of the diagram and the inset relate to the quasi-CW RF excitation field while the lines in the right part of diagram and inset relate to the 2nd harmonic thermoacoustic response from biological tissue.

The measurement of such responses is in stark difference to corresponding measurements employing pulsed light and is assumed to be based on non-linear effects as outlined as follows.

A thermoacoustic response or, upon excitation with light, an optoacoustic response can be derived by observing the photoacoustic wave equation in the frequency domain, i.e.

$$\left(\nabla^2 + \frac{\omega^2}{v_s^2}\right)\hat{p}(\vec{r},\omega) = -j\omega\frac{\beta}{C_p}\left(q\nabla^2\hat{T}(\vec{r},\omega) + \hat{Q}_x(\vec{r},\omega)\right) \quad , \qquad (11)$$

which relates the detected pressure $\hat{p}(\vec{r},\omega)$ to the optical density $\hat{Q}_{opt}(\vec{r},\omega)=\mu_a I(r)\hat{F}(\omega)\eta_{eff}\exp(-\mu_a|z-z_0|)$, whereby $\mu_a$ is the optical absorption coefficient, $\eta_{eff}$ the conversion efficiency, $I$ the intensity at position r, q the thermal conductivity, $v_s$ the speed of sound in matter, e.g. water, $\beta$ the thermal coefficient of cubic expansion, $C_p$ the specific heat capacity, and $T$ the tissue temperature at position r and time point $t$.

Solving Eq. 11 for pressure gives the Fourier domain representation of the pressure wave, i.e.

$$\hat{p}_x(\vec{r},\omega) = -\frac{j\omega\beta}{4\pi C_p}\int_V \frac{\exp\left(jk|\vec{r}-\vec{r}'|\right)}{|\vec{r}-\vec{r}'|}\left[q\nabla^2\hat{T}(\vec{r}',\omega) + \hat{Q}_x(\vec{r}',\omega)\right]d^3\vec{r}' \quad . \qquad (12)$$

Assuming a monochromatic optical stimulation in the frequency domain $f(t) = \cos\left(2\pi f t\right)\xrightarrow{FD}\hat{F}(\omega) = \pi\left[\delta\left(\omega+\omega_0\right)+\delta\left(\omega-\omega_0\right)\right]$, where $F_{(w)}$ relates to the Fourier transform of the modulation frequency in the optical density function and the RF power function, Eq. 12 yields a linear relation between pressure $\hat{p}_{opt}$ and optical power $\hat{Q}_{opt}$ as a function of $\omega$.

In the case of RF excitation, the deposited power function can be described by Poynting's theorem, i.e.,

$$\hat{Q}_{RF}\left(\vec{r},\omega\right) = \frac{\sigma(\vec{r})}{2}\left|\hat{E}\left(\vec{r},\omega\right)\right|^2 + \pi f \varepsilon''(\vec{r})\left|\hat{E}\left(\vec{r},\omega\right)\right|^2 + \pi f \mu''(\vec{r})\left|\hat{H}\left(\vec{r},\omega\right)\right|^2 \qquad (13)$$

where $\varepsilon''$ denotes the imaginary part of the complex permittivity, $\mu''$ is the imaginary part of the complex permeability, $\hat{E}$ is the spectral electric field amplitude and $\hat{H}$ is the spectral magnetic field amplitude. Assuming an electric field $\vec{E}(\vec{r}, t)=E_0\vec{e}_r\cos(\omega t)$ oscillating at frequency $\omega$ (corresponding to the first frequen- cy $f_1$), the power of the electric field can be rewritten in Fourier domain as

$$\left| \hat{E}\left(\vec{r},\omega\right) \right|^2 = E_0{}^2 \vec{e}_r \left[ \delta\left(\omega - 2\omega_0\right) + \delta\left(\omega + 2\omega_0\right) + 2\delta\left(\omega\right) \right] . \qquad (14)$$

Eq. 14 yields a non-linear relation of the RF-induced acoustic pressure $\hat{p}_{RF}$, on the one hand, to the frequency $2\omega$ (corresponding to the second frequency $f_2$) observed in the thermoacoustic response measurements of Fig. 4, on the other hand.

Because the thermoacoustic responses are measured at a frequency $2\omega$ ($f_2$) corresponding to twice the frequency $\omega$ ($f_1$) of the exciting electric field $E(\vec{r},t)=E_0\vec{e}_r\cos(\omega t)$, such measurements can also be regarded as "2nd harmonic" thermoacoustic measurements.

By means of the sensor device and the method described above, according thermoacoustic responses are also obtained upon magnetic excitation of the medium 2 consisting of or comprising, e.g., conductive material, like metals, and/or magnetic solids and/or fluids. As absorption of transient magnetic fields by objects is related to temperature increase, conductive or magnetic compounds exposed to MHz alternating fields emit ultrasonic waves upon excitation. When exposed to alternating magnetic fields, eddy currents are induced within conductive matter leading to heating, which depends on the conductivity, field frequency and the magnetic flux. Therefore, after substituting the oscillating electric field $E(r, t)$ by an alternating magnetic field $H(r, t)$, equations (11) to (14) accordingly apply to magnetic fields, wherein the power of the magnetic field in Fourier domain can be written as

$$\left| \hat{H}\left(\vec{r},\omega\right) \right|^2 = \frac{\pi}{2} H_0{}^2 \vec{e}_r \left[ \delta\left(\omega - 2\omega_0\right) + \delta\left(\omega + 2\omega_0\right) + 2\delta\left(\omega\right) \right] . \qquad (15)$$

For example, exciting a conducting absorber, like chrome vanadium steel, as the medium 2 with an alternating magnetic field at a first frequency $f_1$ of approximately 780 kHz results in the generation of a thermoacoustic response at a second frequency $f_2$ of about 1.56 MHz. Moreover, non-linear ultrasonic responses are also present in media 2 which contain other than conductive metal structures. For example, $Fe_3O_4$ magnetic iron oxide nanoparticles (fluidMAG-D 50 nm, fluidMAG-D 100 nm, chemicell GmbH, Berlin, Germany) employed in magnetic hyperthermia and MRI are exposed to AC magnetic fields of first frequencies $f_1$ = 780 kHz and 1.24 MHz and strength B $\approx$ 5 mT. Preferably, the heating rate of water serves as the baseline measurement, because water does not respond to the magnetic field and therefore has a constant temperature over time, independent of frequency. Also in these cases, non-linear acoustic waves at second frequencies of $f_1$ = 1.56 MHz and 2.48 MHz, respectively, are emitted from the magnetic nanoparticles of the medium 2 in response to alternating magnetic energy deposition.

Figure 8 shows an example of an according sensor unit together with detected thermoacoustic responses of a conductive medium (metal) and magnetic medium (magnetic nanoparticles) to alternating magnetic fields: A. Magnetic induction system for generating an alternating magnetic field comprises a Helmholtz coil or a solenoid; B. Helmholtz coil with metallic absorber as the sensor medium and PZT transducer for detecting non-linear 2nd harmonic acoustic response; C. Spectral measurement of the RF interference (lower peak at 1.56 MHZ) distorting the PZT transducer, and 2nd harmonic acoustic signal (higher peak at 1.56 MHz) from the metallic rod; D. Solenoid used for acoustic measurements with the sensor medium, i.e. magnetic nanoparticles and the acoustic detector, i.e. a FBG; E. close-up image of the solenoid, delineating the measurement and detection geometry; F. Temperature monitoring of magnetic nanoparticles fluid-MAG-D 50 nm, fluidMAG-D 100 nm, nanomag-D-spio and water over a time period of 5 minutes at two different frequencies; and G. 2nd harmonic thermoacoustic response of $Fe_3O_4$ (peak) at approximately 2.3208 MHz (alternating magnetic field of approximately 1.1604 MHz) in comparison with a control measurement consisting of a water tubing (background noise).

The findings set forth above show that 2nd harmonic thermoacoustic detection is an advantageous method for measuring electromagnetic and/or magnetic energy deposition within matter, in particular tissue. The invention can be applied, e.g., to investigate cell-phone energy absorption by human tissues such as the brain as the herein employed power of $P_{ave}$ = 5 W only exceeded the power levels of cell phones by a factor of 2.5 in average. Preferably, a 2nd harmonic detection of thermoelastic responses is employed to reliably measure energy deposition in tissues, in contrast to today's standard which is based on complex simulations employing numerical algorithms or the specific absorption rate (SAR) which defines the absorption of EM power per volume. Implementation of the invention in a tomographic arrangement is possible to provide high-resolution mapping of electric or magnetic absorption.

In order to enable particularly reliable and sensitive measurements of the thermoacoustic responses, the detector 3 (see figures 1 to 3) preferably comprises an ultrasensitive broadband optical-fiber-based interferometric detector, which offers clear signal responses and is impervious to possible electromagnetic interferences. In contrast to conventional interferometry based sensors, the preferred interferometric detector achieves high dynamic range of 106.5 dB which is required to sense the predicted acoustic waves with a sharp pure acoustic frequency peak exhibiting an SNR of 6.1 dB, even at low magnetic fields of approximately 5 mT, cleared of any possible RF interferences. This will be described in more detail in the following.

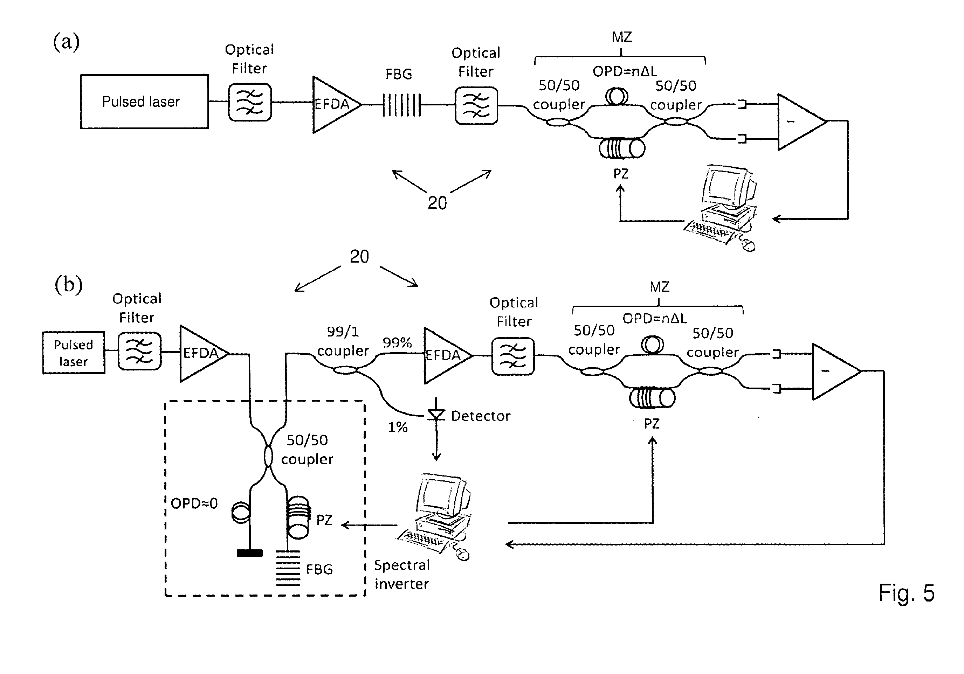

Figure 5 shows examples of setups of an interferometric detector 20 used for pulse-interferometry interrogation of a, preferably $\pi$-phase shifted, fiber bragg grating FBG, wherein with the setup according to fig. 5 (a) variations of the resonance frequency of light transmitted by the FBG, whereas with the setup according to fig. 5 (b) variations of the resonance frequency of light reflected by the FBG are determined.

The interferometric detectors 20 comprise a FBG acting as an interferometric optical filter (fig. 5 (a)) or reflector (fig. 5 (b)), which transmits or blocks certain wavelengths. The transmitted or reflected wavelengths, in particular the resonance frequency, depend on strain or pressure applied to the FBG, so that the FBG is sensitive to pressure variations and hence (ultra-)sound waves.

Detecting acoustic, in particular ultrasound, waves generated in a medium by means of optical interferometry and/or FBGs allows for an accurate and reliable measurement of thermoacoustic responses of the medium, in particular to CW excitation of the medium in a broad frequency range reaching from several Kilohertz (kHz) up to several hundreds of Gigahertz (GHz), i.e. up to the microwave range.

A pulsed laser is provided for generating wideband coherent light pulses. The laser source can be, e.g., a 90 fs laser with a repetition rate of 100 MHz, output power of 60 mW and spectral width of over 100 nm. Preferably, polarization maintaining fibers are used to maintain single polarization.

Preferably, both in the transmission (a) and reflection (b) scheme, variations of the resonance frequency of the FBG are measured using an unbalanced Mach-Zehnder (MZ) interferometer. The MZ interferometer is preferably stabilized to its quadrature point.

Spectral inversion - a technique in which the reflection spectrum is inverted and assumes the shape of the transmission spectrum - can optionally be performed by using a Michelson interferometer (see fig. 5(b)) stabilized to destructive interference on its output arm. "OPD" denotes the optical path difference in the MZ or Michelson interferometer, respectively.

The spectral inversion efficiency can be increased by improving the balance of the Michelson interferometer and by using a narrower, steeper optical band pass filter. By means of spectral inversion, a particularly high visibility can be obtained in the reflection measurement (see fig. 5 (b)).

Preferably, amplifiers, e.g. Erbium-doped fiber amplifiers EDFA, are used to amplify the light pulses of the laser source before feeding same directly (fig. 5 (a)) into the FBG or indirectly via a Michelson interferometer (fig. 5 (b)) into the FBG.

It is, moreover, preferred to filter the light pulses by means of optical filters before feeding same into the EDFA and/or the MZ interferometer. Preferably, the optical filters have a FWHM spectral width of 0.3 nm and were tuned to the frequency of the resonance of the FBG.

A computer-generated feedback signal, fed into piezoelectric fiber stretcher PZ on one of the interferometer arms with a response bandwidth of 100 kHz, can be used to stabilize the interferometers. Preferably, the detection bandwidth corresponds to approximately 20 MHz.

Preferably, the FBG exhibits a bandgap span of 1.38 nm and a FWHM resonance width of 8 pm, corresponding to a coupling coefficient of $\kappa$= 2.58 mm$^{-1}$ and grating length of L = 2.38 mm. These values correspond to spectral-inversion efficiency of 0.66, i.e. the Lorentzian resonance constitutes 66% of the power of *i - rg.*

Preferably, the detector 3 of the sensor unit 1 (see figures 1 to 3) comprises at least the FBG of the interferometric detector 20 shown in fig 5 (a) or (b). Depending on particular application requirements, it may be preferred to also integrate the optical filter/s and/or the EFDA and/or the MZ interferometer and/or the Michelson interferometer into detector 3. One or more of the aforementioned components may also be integrated in a separate constituent of the detector 3 and/or be part of the processing unit 4.

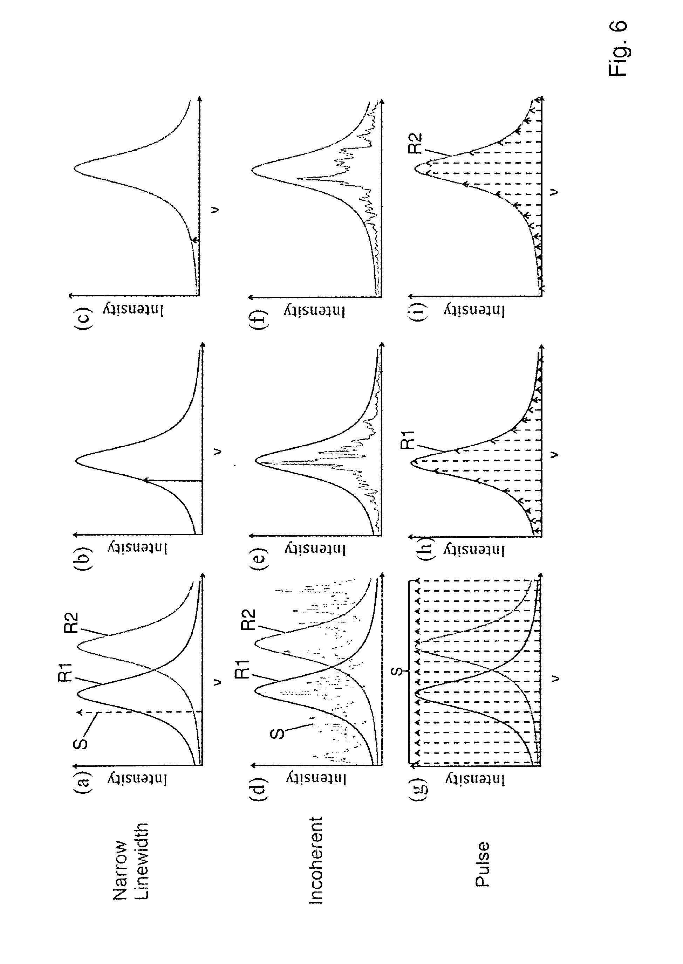

Figure 6 shows a schematic description of (a - c) narrowband interrogation (d - f), wideband CW interrogation and (g - i) wideband pulse interrogation of an optical bandpass filter, preferably the FBG shown in fig. 5 (a) and (b).

The first column of fig. 6 shows examples of a spectrum S of the light source together with Lorenzian shaped resonance spectra R1 and R2 for two central frequencies of an FBG. The two additional columns show examples of the spectrum of the light source after passing through each of the respective resonance spectra R1 and R2. In the following, a qualitative comparison of the three methods shown in fig. 6 will be given, wherein the central optical frequency of the resonance is denoted by $v_0$ and its width by $\Delta v$.

In coherent, narrow linewidth CW interrogation (fig. 6 (a)), the frequency of a narrow linewidth laser v is usually tuned to the point of maximum steepness in the resonance ($v - v_0 = \Delta v/6$) and the reflected or transmitted intensity is monitored. As the resonance shifts, the monitored intensity changes (figs. 6 (b) and 6 (c)). However, shifts that are larger than the width of the resonance can effectively saturate the measurement. Accordingly, in order to lock the laser wavelength to the resonance, e.g. by using negative feedback, the tuning rate should exceed the maximum rate of change of the intensity. As a result, the narrower the resonance, the faster the feedback response would have to be. Despite the ubiquity of this method, its use has been mostly limited to lab conditions due to its vulnerability to environmental factors and the difficulty of multiplexing being a key factor in imaging applications.

Figure 6 (d) illustrates incoherent, wideband interrogation. The transmitted light assumes the spectral shape R1 and R2 of the resonance (see figs. 6 (e) and 6 (f)) and its mean frequency can be monitored, e.g. using a Mach-Zehnder interferometer and demodulation, to detect resonance shifts. Generally, demodulation may be performed either actively or passively. In active demodulation, the interferometer can be locked to a specific state for which its output is a linear function of the monitored resonance frequency. In passive demodulation, optical manipulation is applied to obtain two complementing outputs out of the interferometer which can be processed to monitor the resonance frequency without any locking mechanism. The ability to perform sensor interrogation without the need of a feedback-based compensation mechanism is an inherent advantage of incoherent CW interferometry over coherent CW interferometry and is a direct result of wideband illumination. However, in the case of incoherent CW interferometry, this comes at the cost of lower sensitivity. Because the spectral distribution of the light source's intensity is an inherently random process, the mean frequency of the transmitted light does not exactly coincide with that of the resonance, but rather fluctuates. These fluctuations pose an intrinsic limitation on the detection sensitivity.

In order to overcome the drawbacks of the approaches mentioned above, it is particularly preferred to interrogate the optical resonator, e.g. the FBG, by means of wideband pulse interferometry, wherein pulsed lasers are used which are both wideband and coherent. Preferred laser sources exhibit a wideband comb structure S in the spectral domain as exemplarily shown in fig. 6 (g) with a spacing $\nu_s$ between the comb's teeth being equal to the repetition rate of the laser pulses. By passing through the resonance of an FBG, the envelope of the comb takes up the form of the resonance spectrum R1 and R2 (see figures 6 (h) and (i)), while detection can be performed by estimating the mean frequency of the resulting spectrum. In the time domain, the original laser pulses are broadened as a result of being convolved with the impulse response of the resonance. Thus, in order to assure that the mean frequencies of the resonance and of the filtered comb coincide, it is sufficient to ensure that the broadened pulses of the laser source do not overlap, i.e. $\tau_g \ll \nu_s^{-1}$, where $\tau_g$ is the effective duration of the pulses at the output of the resonator, e.g. the FBG. In other words, the repetition rate $\nu_s$ of the laser pulses has to be significantly smaller, e.g. more than 5 or 10 times smaller, than the reciprocal value of the effective duration $\tau_g$ of the pulses at the output of the resonator, i.e. $\nu_s \ll \tau_g^{-1}$. In such a case, the spectrum of each pulse approximates that of the FBG resonance.

By means of the preferred approach for interrogating high-finesse optical resonators, like FBGs, a high stability against environmental conditions is achieved without compromising sensitivity. Moreover, the use of a wideband source opens the possibility for scalable multiplexing, e.g. in implementations applying low-coherence sources.

**Claims**

1. Sensor device for detecting, in particular imaging, thermoacoustic responses of a medium (2), comprising a detector (3) for detecting acoustic waves, in particular ultrasonic waves, generated in the medium (2) upon absorption of energy from an electromagnetic field (10) and/or from an alternating magnetic field comprising electromagnetic waves or magnetic waves, respectively, of a first frequency ($f_1$),
**characterized in that**
the detector (3) is designed for detecting acoustic waves of a second frequency ($f_2$), wherein the second frequency ($f_2$) is higher than the first frequency ($f_1$).

2. Sensor device according to claim 1, wherein the second frequency ($f_2$) of the detected acoustic waves is equivalent to 1.8 to 2.2 times the first frequency ($f_1$), preferably twice the first frequency ($f_1$).

3. Sensor device according to claim 1 or 2, wherein the sensitivity of the detector (3) exhibits a maximum at a center frequency which is equivalent to 1.5 to 2.5 times the first frequency ($f_1$).

4. Sensor device according to claim 3, wherein the sensitivity of the detector (3) for acoustic waves has a bandwidth around the center frequency, wherein the bandwidth is equivalent to 50 % to 80 % of the center frequency or higher than 80 % of the center frequency.

5. Sensor device according to any of the foregoing claims, wherein the electromagnetic field (10) and/or the alternating magnetic field comprising electromagnetic waves or magnetic waves, respectively, of the first frequency ($f_1$) and/or

at a first frequency band, and wherein the detector (3) has at least one of the following properties:

- the detector is designed for detecting acoustic waves at the fundamental frequency of the electromagnetic field (10) and/or magnetic field and/or odd harmonics of the fundamental frequency;
- the detector is designed for detecting acoustic waves at a second frequency band, wherein the second frequency band contains higher frequency components and/or harmonics of the first frequency band including the fundamental frequency.

6. Sensor device according to any of the foregoing claims, wherein the detector (3) comprises an optical interferometry detector (20) for detecting the acoustic waves, in particular the ultrasonic waves, by means of optical interferometry.

7. Sensor device according to claim 6, wherein the optical interferometry detector (20) comprises a wideband light source generating pulsed light.

8. Sensor device according to claim 7, the wideband light source generating coherent pulsed light.

9. Sensor device according to any of the foregoing claims, wherein the detector (3) comprises a fiber bragg grating (FBG) element.

10. Sensor device according to claim 9, the fiber bragg grating element (FBG) being a $\pi$-phase-shifted fiber bragg grating element.

11. Sensor device according to any of the foregoing claims, wherein the first frequency ($f_1$) is in the range between 3 kHz and 300 GHz, in particular between 400 MHz and 2 GHz.

12. Sensor device according to any of the foregoing claims, wherein the electromagnetic field (10) or magnetic field, respectively, is a field of continuous waves (CW) or of quasi-continuous waves (quasi-CW).

13. Sensor device according to any of the foregoing claims, comprising a processing unit (4) for deriving from the at least one detected acoustic wave one or more properties relating to the medium (2), in particular electric and/or dielectric and/or magnetic properties of the medium (2), like conductivity, permittivity and/or susceptibility.

14. Sensor device according to any of the foregoing claims, comprising a processing unit (4) for deriving from the at least one detected acoustic wave one or more properties relating to the electromagnetic field (10) or magnetic field, respectively, in particular the strength and/or frequency and/or spatial distribution and/or polarization of the electromagnetic field (10) or magnetic field, respectively.

15. Sensor device according to any of the foregoing claims comprising a processing unit (4) for deriving from the at least one detected acoustic wave one or more properties relating to the absorption or deposition of energy from the electromagnetic field (10) and/or magnetic field, respectively, in the medium (2).

16. Method for detecting, in particular imaging, thermoacoustic responses of a medium (2), comprising detecting acoustic waves, in particular ultrasonic waves, generated in the medium (2) upon absorption of energy from an electromagnetic field (10) and/or from an alternating magnetic field comprising electromagnetic waves or magnetic waves, respectively, of a first frequency ($f_1$),
**characterized by**
detecting acoustic waves of a second frequency ($f_2$), wherein the second frequency ($f_2$) is higher than the first frequency ($f_1$).

Fig. 1

Fig. 2

Fig. 3

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 13 00 3754

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EMERSON J F ET AL: "Electromagnetic acoustic imaging", IEEE TRANSACTIONS ON ULTRASONICS, FERROELECTRICS AND FREQUENCY CONTROL, IEEE, US, vol. 60, no. 2, 1 February 2013 (2013-02-01), pages 364-372, XP011505902, ISSN: 0885-3010, DOI: 10.1109/TUFFC.2013.2572 | 1-5, 11-16 | INV. G01N29/24 G01N29/265 G01N29/22 A61B5/00 G01S15/89 |
| Y | * abstract; figure 1 * * page 365, left-hand column, last paragraph - page 367, left-hand column, paragraph 1 * | 6-10 | |
| Y | US 2011/040176 A1 (RAZANSKY DANIEL [DE] ET AL) 17 February 2011 (2011-02-17) * abstract; figure 1 * * paragraphs [0015] - [0040] * | 6-8 | |
| Y | WO 2006/061829 A1 (GLUCON INC [US]; PESACH BENNY [IL]; NAGAR RON [IL]; BITTON GABRIEL [IL] 15 June 2006 (2006-06-15) * abstract; figure 1 * * paragraph [0021] - paragraph [0050] * | 9,10 | TECHNICAL FIELDS SEARCHED (IPC) G01N A61B G01S |

The present search report has been drawn up for all claims

| Place of search | Date of completion of the search | Examiner |
|---|---|---|
| Munich | 27 January 2014 | Uttenthaler, Erich |

EPO FORM 1503 03.82 (P04C01)

**ANNEX TO THE EUROPEAN SEARCH REPORT
ON EUROPEAN PATENT APPLICATION NO.**

EP 13 00 3754

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2014

| Patent document cited in search report | | Publication date | Patent family member(s) | | Publication date |
|---|---|---|---|---|---|
| US 2011040176 | A1 | 17-02-2011 | EP 2110076 | A1 | 21-10-2009 |
| | | | EP 2254467 | A1 | 01-12-2010 |
| | | | JP 2011512180 | A | 21-04-2011 |
| | | | US 2011040176 | A1 | 17-02-2011 |
| | | | WO 2009103502 | A1 | 27-08-2009 |
| WO 2006061829 | A1 | 15-06-2006 | NONE | | |

EPO FORM P0459

For more details about this annex : see Official Journal of the European Patent Office, No. 12/82